# EUROPEAN PATENT APPLICATION

(11) **EP 4 495 119 A1**
(43) Date of publication of application: **22.01.2025**
(21) Application number: 23769876.6
(22) Date of filing: 16.03.2023
(51) Int. Cl.: C07D 471/04

(54) **FUSED HETEROCYCLIC COMPOUND, AND PREPARATION METHOD THEREFOR AND MEDICAL USE THEREOF**

(30) Priority: 16.03.2022 CN 202210257186
(71) Applicant: Jiangsu Hengrui Pharmaceuticals Co., Ltd., Lianyungang, Jiangsu 222047 (CN); Shanghai Hengrui Pharmaceutical Co., Ltd., Shanghai 200245 (CN)
(72) Inventor: ZHANG, Rui, Shanghai 200245 (CN); LI, Xin, Shanghai 200245 (CN); ZHANG, Jing, Shanghai 200245 (CN); YUAN, Huiqing, Shanghai 200245 (CN); HE, Feng, Shanghai 200245 (CN); TAO, Weikang, Shanghai 200245 (CN)
(74) Representative: Potter Clarkson
(86) International application number: PCT/CN2023/081908
(87) International publication number: WO 2023/174374

(57) **Abstract**

The present disclosure relates to a fused heterocyclic compound, and a preparation method therefor and a medical use thereof. Specifically, the present disclosure relates to a fused heterocyclic compound shown in general formula (I), a preparation method for the compound, a pharmaceutical composition containing the compound, and a use of the compound as an RIPK1 kinase inhibitor, in particular a use in the preparation of a drug for treating and/or preventing RIPK1 kinase-mediated diseases or conditions.

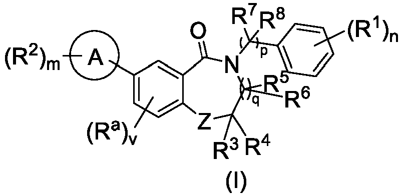

## Description

### TECHNICAL FIELD

The present disclosure belongs to the field of pharmaceutics, and relates to a fused heterocyclic compound, a preparation method therefor, and pharmaceutical use thereof. Specifically, the present disclosure relates to a fused heterocyclic compound of general formula (I), a preparation method thereof, a pharmaceutical composition comprising the compound, and use thereof as a RIPK1 kinase inhibitor, particularly use thereof in the preparation of a medicament for treating and/or preventing a RIPK1 kinase-mediated disease or disorder.

### BACKGROUND

Necroptosis is a programmed cell death, and unlike apoptosis, necroptosis can cause the rupture of cell membranes, resulting in the release of damage-associated molecular patterns (DAMPs), activating immune cells, and thus leading to a strong immune response. The activated immune cells release inflammatory cytokines such as TNF-α and the like, which in turn accelerates the necroptosis of cells, finally, causing tissue damage. The molecular biological mechanism by which necroptosis occurs is that RIPK1/RIPK3 phosphorylates MLKL, and MLKL is subjected to multimerization and accumulates on the cell membrane to cause membrane rupture, resulting in cell death.

Activation of RIPK1 kinase activity can induce the necroptosis of cells and is associated with a variety of human autoimmune diseases and neurodegenerative diseases. When cells are stimulated by cytokines such as TNF and the like, death receptors are activated, and RIPK1 is recruited to cell membranes. Then, RIPK1 is ubiquitinated and recruits other proteins to form complex I protein complex. The complex I protein complex can activate NF-κB pathways and promote cell survival. In some cases, for example, when the ubiquitination of RIPK1 is inhibited, RIPK1 is subjected to autophosphorylation, activating its own activity and inducing cell death. When the activated protease caspase 8 exists, RIPK1-dependent apoptosis of cells occurs; when the protease caspase 8 is not activated, necroptosis of cells occurs. The survival or death of cells is precisely regulated by the levels of RIPK1 phosphorylation and ubiquitination. Notably, there are many negative regulators of RIPK1 in cells, which prevent over-activation of RIPK1. Dysfunction of these negative regulators can induce a variety of diseases. For example, dysfunction of OPTN can induce amyotrophic lateral sclerosis (ALS or Lou Gehrig's disease or gradual freezing disease).

It is safe to inhibit the kinase activity of RIPK1, and this inhibition exhibits an excellent therapeutic effect in a variety of disease models. Genetic studies have shown that RIPK1 double-knockout mice died 1-3 days after birth, but RIPK1 D138N, etc. kinase-dead knock-in mice showed normal phenotypes, suggesting that it is safe to inhibit the kinase activity of RIPK1. Genetic or compound inhibition of the kinase activity of RIPK1 exhibits excellent efficacy in a variety of disease models. These diseases include ischemic diseases such as ischemic heart disease, acute kidney injury, ischemic brain injury, etc.; autoimmune diseases such as rheumatoid arthritis, psoriasis, inflammatory bowel disease, etc.; inflammatory diseases such as systemic inflammatory response syndrome, pancreatitis, hepatitis, etc.; neurodegenerative diseases such as amyotrophic lateral sclerosis, multiple sclerosis, Alzheimer's disease, Parkinson's disease, etc.

Related published patent applications include WO2020192562A1, WO2018148626A1, WO2021160109A1, WO2014125444A1, WO2016027253A1, WO2017109724A1, WO2017004500A1, CN113773316A, WO2013006485A1, WO2017136727A2, etc.

### SUMMARY

The present disclosure aims to provide a compound of general formula (I) or a pharmaceutically acceptable salt thereof: 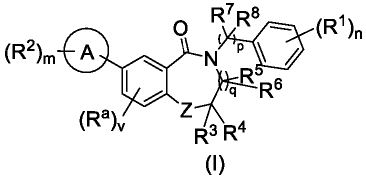 wherein:
ring A is selected from the group consisting of heteroaryl, cycloalkyl, heterocyclyl and polycyclic aryl;
Z is selected from the group consisting of -C(O)-, -S(O)ᵣ, -NRⁿ¹-, -CR^{z1}R^{z2}- and -O-;
each R¹ is identical or different and is independently selected from the group consisting of halogen, alkyl, alkenyl, alkynyl, nitro, cyano, -NRⁿ²Rⁿ³, -C(O)NRⁿ⁴Rⁿ⁵, -NRⁿ⁶C(O)R^{9a}, -C(O)R^{9b}, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
each R² is identical or different and is independently selected from the group consisting of halogen, alkyl, alkenyl, alkynyl, nitro, cyano, oxo, -NRⁿ²Rⁿ³, -C(O)NRⁿ⁴Rⁿ⁵, - NRⁿ⁶C(O)R^{9a}, -C(O)R⁹⁶, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
each R^{a} is identical or different and is independently selected from the group consisting of halogen, alkyl, alkenyl, alkynyl, nitro, cyano, -NRⁿ²Rⁿ³, -C(O)NRⁿ⁴Rⁿ⁵, -NRⁿ⁶C(O)R^{9a}, -C(O)R^{9b}, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
R³ and R⁴ are identical or different and are each independently selected from the group consisting of a hydrogen atom, a deuterium atom, halogen, hydroxy, carboxyl, alkyl, haloalkyl, hydroxyalkyl, alkenyl, alkynyl, nitro, cyano, -NRⁿ²Rⁿ³, -C(O)NRⁿ⁴Rⁿ⁵, - C(O)R^{9b}, -OC(O)R^{9c}, -C(O)OR^{10a}, -OR^{10b}, cycloalkyl, heterocyclyl, aryl and heteroaryl; or R³ and R⁴, together with the carbon atom to which they are attached, form cycloalkyl or heterocyclyl;
R⁵ and R⁶ are identical or different and are each independently selected from the group consisting of a hydrogen atom, a deuterium atom, halogen, hydroxy, carboxyl, alkyl, haloalkyl, hydroxyalkyl, alkenyl, alkynyl, nitro, cyano, -NRⁿ²Rⁿ³, -OR^{10b}, cycloalkyl, heterocyclyl, aryl and heteroaryl;
R⁷ and R⁸ are identical or different and are each independently selected from the group consisting of a hydrogen atom, a deuterium atom, halogen, hydroxy, carboxyl, alkyl, haloalkyl, hydroxyalkyl, alkenyl, alkynyl, nitro, cyano, -NRⁿ²Rⁿ³, -OR^{10b}, cycloalkyl, heterocyclyl, aryl and heteroaryl; or R⁷ and R⁸, together with the carbon atom to which they are attached, form cycloalkyl or heterocyclyl;
R^{z1} and R^{z2} are identical or different and are each independently selected from the group consisting of a hydrogen atom, halogen, hydroxy, carboxyl, alkyl, haloalkyl, hydroxyalkyl, alkenyl, alkynyl, nitro, cyano, -NRⁿ²Rⁿ³, -C(O)NRⁿ⁴Rⁿ⁵, -C(O)R⁹⁶, - OC(O)R^{9c}, -C(O)OR^{10a}, -OR^{10b}, cycloalkyl, heterocyclyl, aryl and heteroaryl; or R^{z1} and R^{z2}, together with the carbon atom to which they are attached, form cycloalkyl or heterocyclyl;
Rⁿ¹, Rⁿ², Rⁿ³ , Rⁿ⁴, Rⁿ⁵ , Rⁿ⁶ , Rⁿ⁷ and Rⁿ⁸ are identical or different at each occurrence and are each independently selected from the group consisting of a hydrogen atom, alkyl, haloalkyl, hydroxyalkyl, cycloalkyl, heterocyclyl, aryl and heteroaryl; or (Rⁿ² and Rⁿ³), (Rⁿ⁴ and Rⁿ⁵), and (Rⁿ⁷ and Rⁿ⁸), together with the nitrogen atom to which they are attached, each form heterocyclyl, wherein each heterocyclyl is independently optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl and heteroaryl;
R^{9a}, R^{9b}, R^{9c}, R^{10a}, R^{10b} and R^{10c} are identical or different at each occurrence and are each independently selected from the group consisting of a hydrogen atom, alkyl, hydroxyalkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
m is 0, 1, 2, 3, 4, 5 or 6;
n is 2, 3, 4 or 5;
p is 0, 1 or 2;
q is 1, 2 or 3;
r is 0, 1 or 2;
t is 0, 1 or 2; and
v is 0, 1, 2 or 3.

In some embodiments of the present disclosure, provided is the compound of general formula (I) or the pharmaceutically acceptable salt thereof, wherein q is 1 or 2; preferably, q is 1.

In some embodiments of the present disclosure, provided is the compound of general formula (I) or the pharmaceutically acceptable salt thereof, wherein p is 0 or 1; preferably, p is 1.

In some embodiments of the present disclosure, the compound of general formula (I) or the pharmaceutically acceptable salt thereof is a compound of general formula (II) or a pharmaceutically acceptable salt thereof: 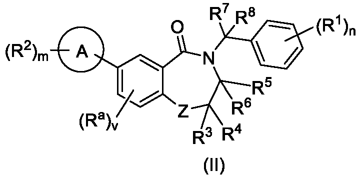 wherein:
ring A, R¹ to R⁸, R^{a}, v, Z, n and m are as defined in general formula (I).

In some embodiments of the present disclosure, provided is the compound of general formula (I) or general formula (II) or the pharmaceutically acceptable salt thereof, wherein R⁵ and R⁶ are identical or different and are each independently selected from the group consisting of a hydrogen atom, a deuterium atom and C₁₋₆ alkyl; preferably, R⁵ and R⁶ are identical or different and are each independently a hydrogen atom or C₁₋₆ alkyl; and more preferably, R⁵ and R⁶ are both hydrogen atoms.

In some embodiments of the present disclosure, provided is the compound of general formula (I) or general formula (II) or the pharmaceutically acceptable salt thereof, wherein R⁷ and R⁸ are identical or different and are each independently selected from the group consisting of a hydrogen atom, a deuterium atom and C₁₋₆ alkyl; preferably, R⁷ and R⁸ are identical or different and are each independently a hydrogen atom or C₁₋₆ alkyl; and more preferably, R⁷ and R⁸ are both hydrogen atoms.

In some embodiments of the present disclosure, the compound of general formula (I) or general formula (II) or the pharmaceutically acceptable salt thereof is a compound of general formula (III) or a pharmaceutically acceptable salt thereof: 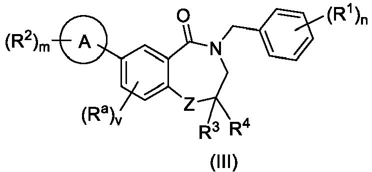 wherein:
ring A, R¹ to R⁴, R^{a}, v, Z, n and m are as defined in general formula (I).

In some embodiments of the present disclosure, provided is the compound of general formula (I), general formula (II) or general formula (III) or the pharmaceutically acceptable salt thereof, wherein R³ and R⁴ are identical or different and are each independently selected from the group consisting of a hydrogen atom, a deuterium atom and C₁₋₆ alkyl, or R³ and R⁴, together with the carbon atom to which they are attached, form 3- to 6-membered cycloalkyl or 3- to 6-membered heterocyclyl; preferably, R³ and R⁴ are identical or different and are each independently a hydrogen atom or C₁₋₆ alkyl, or R³ and R⁴, together with the carbon atom to which they are attached, form 3- to 6-membered cycloalkyl; and more preferably, R³ and R⁴ are both hydrogen atoms.

In some embodiments of the present disclosure, provided is the compound of general formula (I), general formula (II) or general formula (III) or the pharmaceutically acceptable salt thereof, wherein n is 2 or 3; and each R¹ is identical or different and is independently selected from the group consisting of halogen, C₁₋₆ alkyl, C₁₋₆ alkoxy, C₁₋₆ haloalkyl and C₁₋₆ haloalkoxy; preferably, n is 2; and two R¹ are identical or different and are each independently selected from the group consisting of halogen, C₁₋₆ alkyl, C₁₋₆ alkoxy, C₁₋₆ haloalkyl and C₁₋₆ haloalkoxy; more preferably, n is 2; and two R¹ are identical or different and are each independently halogen or C₁₋₆ haloalkoxy; further preferably, n is 2; and one of the two R¹ is halogen and the other is C₁₋₆ haloalkoxy; and most preferably, n is 2; and one of the two R¹ is a fluorine atom and the other is trifluoromethoxy.

In some embodiments of the present disclosure, the compound of general formula (I), general formula (II) or general formula (III) or the pharmaceutically acceptable salt thereof is a compound of general formula (IV) or a pharmaceutically acceptable salt thereof: 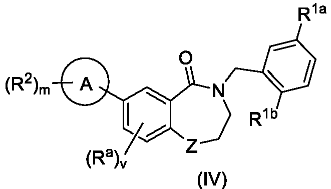 wherein:
R^{1a} and R^{1b} are identical or different and are each independently selected from the group consisting of halogen, alkyl, alkenyl, alkynyl, nitro, cyano, -NRⁿ²Rⁿ³, -C(O)NRⁿ⁴Rⁿ⁵, - NRⁿ⁶C(O)R^{9a}, -C(O)R⁹⁶, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
ring A, R², R^{a}, v, Z, Rⁿ², Rⁿ³, Rⁿ⁴, Rⁿ⁵, Rⁿ⁶, Rⁿ⁷, Rⁿ⁸, R^{9a}, R^{9b}, R^{9c}, R^{10a}, R^{10b}, R^{10c}, t and m are as defined in general formula (I).

In some embodiments of the present disclosure, the compound of general formula (I), general formula (II), general formula (III) or general formula (IV) or the pharmaceutically acceptable salt thereof is a compound of general formula (V) or a pharmaceutically acceptable salt thereof: 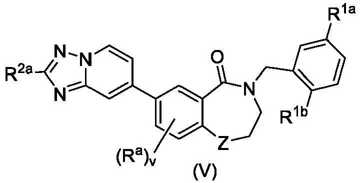 wherein:
R^{2a} is selected from the group consisting of halogen, alkyl, alkenyl, alkynyl, nitro, cyano, -NRⁿ²Rⁿ³, -C(O)NRⁿ⁴Rⁿ⁵, -NRⁿ⁶C(O)R^{9a}, -C(O)R^{9b}, -OC(O)R^{9c}, -C(O)OR^{10a}, -OR^{10b}, - S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
R^{1a} and R^{1b} are identical or different and are each independently selected from the group consisting of halogen, alkyl, alkenyl, alkynyl, nitro, cyano, -NRⁿ²Rⁿ³, -C(O)NRⁿ⁴Rⁿ⁵, - NRⁿ⁶C(O)R^{9a}, -C(O)R⁹⁶, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
R^{a}, v, Z, Rⁿ², Rⁿ³, Rⁿ⁴ , Rⁿ⁵, Rⁿ⁶, Rⁿ⁷ , Rⁿ⁸, R^{9a}, R^{9b}, R^{9c}, R^{10a}, R^{10b}, R^{10c} and t are as defined in general formula (I).

In some embodiments of the present disclosure, provided is the compound of general formula (IV) or general formula (V) or the pharmaceutically acceptable salt thereof, wherein R^{1a} and R^{1b} are identical or different and are each independently selected from the group consisting of halogen, C₁₋₆ alkyl, C₁₋₆ alkoxy, C₁₋₆ haloalkyl and C₁₋₆ haloalkoxy; preferably, R^{1a} and R^{1b} are identical or different and are each independently halogen or C₁₋₆ haloalkoxy; more preferably, R^{1a} is C₁₋₆ haloalkoxy, and R^{1b} is halogen; and most preferably, R^{1a} is trifluoromethoxy, and R^{1b} is a fluorine atom.

In some embodiments of the present disclosure, provided is the compound of general formula (V) or the pharmaceutically acceptable salt thereof, wherein R^{2a} is selected from the group consisting of a hydrogen atom, halogen, C₁₋₆ alkyl, C₁₋₆ alkoxy, C₁₋₆ haloalkyl, C₂₋₆ alkenyl, C₂₋₆ alkynyl, C₁₋₆ haloalkoxy, -NRⁿ²Rⁿ³ and -NRⁿ⁶C(O)R^{9a}; Rⁿ², Rⁿ³ and Rⁿ⁶ are identical or different and are each independently a hydrogen atom or C₁₋₆ alkyl; R^{9a} is selected from the group consisting of C₁₋₆ alkyl, C₁₋₆ hydroxyalkyl, 3- to 6-membered cycloalkyl and 3- to 6-membered heterocyclyl; preferably, R^{2a} is -NRⁿ²Rⁿ³ or - NRⁿ⁶C(O)R^{9a}; Rⁿ², Rⁿ³ and Rⁿ⁶ are identical or different and are each independently a hydrogen atom or C₁₋₆ alkyl; R^{9a} is C₁₋₆ alkyl; more preferably, R^{2a} is -NRⁿ²Rⁿ³; Rⁿ² and Rⁿ³ are identical or different and are each independently a hydrogen atom or C₁₋₆ alkyl; and most preferably, R^{2a} is -NH₂.

In some embodiments of the present disclosure, provided is the compound of general formula (I), general formula (II), general formula (III) or general formula (IV) or the pharmaceutically acceptable salt thereof, wherein each R² is identical or different and is independently selected from the group consisting of halogen, C₁₋₆ alkyl, C₁₋₆ alkoxy, C₁₋₆ haloalkyl, C₂₋₆ alkenyl, C₂₋₆ alkynyl, C₁₋₆ haloalkoxy, -NRⁿ²Rⁿ³ and -NRⁿ⁶C(O)R^{9a}; Rⁿ², Rⁿ³ and Rⁿ⁶ are identical or different and are each independently a hydrogen atom or C₁₋₆ alkyl, and R^{9a} is selected from the group consisting of C₁₋₆ alkyl, C₁₋₆ hydroxyalkyl, 3-to 6-membered cycloalkyl and 3- to 6-membered heterocyclyl; and m is 0, 1, 2, 3 or 4; preferably, each R² is identical or different and is independently selected from the group consisting of halogen, C₁₋₆ alkyl, C₁₋₆ alkoxy, C₁₋₆ haloalkyl, C₁₋₆ haloalkoxy, -NRⁿ²Rⁿ³ and -NRⁿ⁶C(O)R^{9a}; Rⁿ², Rⁿ³ and Rⁿ⁶ are identical or different and are each independently a hydrogen atom or C₁₋₆ alkyl, and R^{9a} is C₁₋₆ alkyl or 3- to 6-membered cycloalkyl; and m is 0, 1, 2, 3 or 4; further preferably, each R² is identical or different and is independently -NRⁿ²Rⁿ³ or -NRⁿ⁶C(O)R^{9a}; Rⁿ², Rⁿ³ and Rⁿ⁶ are identical or different and are each independently a hydrogen atom or C₁₋₆ alkyl, and R^{9a} is C₁₋₆ alkyl or 3- to 6-membered cycloalkyl; and m is 0, 1 or 2; still further preferably, R² is -NRⁿ²Rⁿ³ or -NRⁿ⁶C(O)R^{9a}; Rⁿ², Rⁿ³ and Rⁿ⁶ are identical or different and are each independently a hydrogen atom or C₁₋₆ alkyl, and R^{9a} is C₁₋₆ alkyl; and m is 0 or 1; more preferably, R² is -NRⁿ²Rⁿ³; Rⁿ² and Rⁿ³ are identical or different and are each independently a hydrogen atom or C₁₋₆alkyl; and m is 1; and most preferably, R² is -NH₂, and m is 1.

In some embodiments of the present disclosure, provided is the compound of general formula (I), general formula (II), general formula (III) or general formula (IV) or the pharmaceutically acceptable salt thereof, wherein ring A is selected from the group consisting of 5- or 6-membered monocyclic heteroaryl, 8- to 10-membered polycyclic heteroaryl and 8- to 10-membered polycyclic aryl; preferably, ring A is selected from the group consisting of pyridinyl, benzimidazolyl, benzoxazolyl, benzothiazolyl, pyrrolotriazinyl, 5,6,7,8-tetrahydro-triazolopyrazinyl, imidazopyridazinyl and [1,2,4]triazolo[1,5-a]pyridinyl; and more preferably, ring A is [1,2,4]triazolo[1,5-a]pyridinyl.

In some embodiments of the present disclosure, provided is the compound of general formula (I), general formula (II), general formula (III) or general formula (IV) or the pharmaceutically acceptable salt thereof, wherein 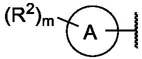 is 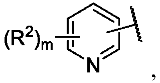 wherein R² and m are as defined in general formula (I); preferably, 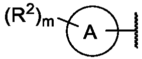 is selected from the group consisting of 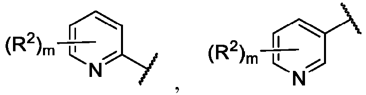 and 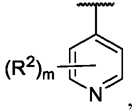 wherein R² and m are as defined in general formula (I).

In some embodiments of the present disclosure, provided is the compound of general formula (I), general formula (II), general formula (III) or general formula (IV) or the pharmaceutically acceptable salt thereof, wherein 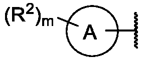 is selected from the group consisting of 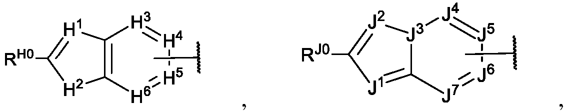 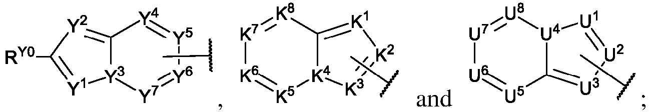
wherein H¹ is a nitrogen atom or CR^{H1}; H² is selected from the group consisting of NR^{H03}, an oxygen atom, a sulfur atom and CR^{H2}; H³ is a nitrogen atom or CR^{H3}; H⁴ is a nitrogen atom or CR^{H4}; H⁵ is a nitrogen atom or CR^{H5}; H⁶ is a nitrogen atom or CR^{H6}; and H³, H⁴, H⁵ and H⁶ are not all nitrogen atoms;
J¹ is a nitrogen atom or CR^{J1}; J² is a nitrogen atom or CR^{J2}; J³ is a nitrogen atom or CR^{J3}; J⁴ is a nitrogen atom or CR^{J4}; J⁵ is a nitrogen atom or CR^{J5}; J⁶ is a nitrogen atom or CR^{J6}; J⁷ is a nitrogen atom or CR^{J7}; and J³, J⁴, J⁵, J⁶ and J⁷ are not all nitrogen atoms; and at least one of J¹, J², 1², J⁴, J⁵, J⁶ and J⁷ is a nitrogen atom;
Y¹ is a nitrogen atom or CR^{Y1}. Y² is a nitrogen atom or CR^{Y2}; Y³ is a nitrogen atom or CR^{Y3}; Y⁴ is a nitrogen atom or CR^{Y4}; Y⁵ is a nitrogen atom or CR^{Y5}; Y⁶ is a nitrogen atom or CR^{Y6}; Y⁷ is a nitrogen atom or CR^{Y7}; and Y³, Y⁴, Y⁵, Y⁶ and Y⁷ are not all nitrogen atoms; and at least one of Y¹, Y², Y³, Y⁴, Y⁵, Y⁶ and Y⁷ is a nitrogen atom;
K¹ is a nitrogen atom or CR^{K1}; K² is a nitrogen atom or CR^{K2}; K³ is a nitrogen atom or CR^{K3}; K⁴ is a nitrogen atom or CR^{K4}; K⁵ is a nitrogen atom or CR^{K5}; K⁶ is a nitrogen atom or CR^{K6}; K⁷ is a nitrogen atom or CR ^{K7} ; K⁸ is a nitrogen atom or CR^{K8}; and K⁴, K⁵, K⁶, K⁷ and K⁸ are not all nitrogen atoms; and at least one of K¹, K², K³, K⁴, K⁵, K⁶, K⁷ and K⁸ is a nitrogen atom;
U¹ is a nitrogen atom or CR^{U1}; U² is a nitrogen atom or CR^{U2}; U³ is a nitrogen atom or CR^{U3}; U⁴ is a nitrogen atom or CR^{U4}; U⁵ is a nitrogen atom or CR^{U5}; U⁶ is a nitrogen atom or CR^{U6}; U⁷ is a nitrogen atom or CR^{U7}; U⁸ is a nitrogen atom or CR^{U8}; and U⁴, U⁵, U⁶, U⁷ and U⁸ are not all nitrogen atoms; and at least one of U¹, U², U³, U⁴, U⁵, U⁶, U⁷ and U⁸ is a nitrogen atom;
R^{H0}, R^{J0}, R^{Y0}, R^{H1}, R^{H2}, R^{H3}, R^{H4}, R^{H5}, R^{H6}, R^{J1}, R^{J2}, R^{J3}, R^{J4}, R^{J5} , R^{J6} , R^{J7}, R^{Y1}, R^{Y2}, R^{Y3}, R^{Y4}, R^{Y5}, R^{Y6}, R^{Y7}, R^{U1}, R^{U2}, R^{U3}, R^{U4}, R^{U5}, R^{U6}, R^{U7}, R^{U8}, R^{K1}, R^{K2}, R^{K3}, R^{K4}, R^{K5}, R^{K6}, R^{K7} and R^{K8} are identical or different and are each independently selected from the group consisting of a hydrogen atom, halogen, alkyl, alkenyl, alkynyl, nitro, cyano, -NRⁿ²Rⁿ³, - C(O)NRⁿ⁴Rⁿ⁵, -NRⁿ⁶C(O)R^{9a}, -C(O)R⁹⁶, -OC(O)R^{9c}, -C(O)OR^{10a}, -OR^{10b}, -S(O)ₜR^{10c}, cycloalkyl, heterocyclyl, aryl and heteroaryl; wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
R^{H03} is a hydrogen atom or C₁₋₆ alkyl; t, Rⁿ², Rⁿ³, Rⁿ⁴, Rⁿ⁵, Rⁿ⁶, Rⁿ⁷, Rⁿ⁸, R^{9a}, R^{9b}, R^{9c}, R^{10a}, R^{10b} and R^{10c} are as defined in general formula (I);
preferably, R^{H0}, R^{J0} and R^{Y0} are identical or different and are each independently selected from the group consisting of a hydrogen atom, -NRⁿ²Rⁿ³ and -NRⁿ⁶C(O)R^{9a}; R^{H1}, R^{H2}, R^{H3}, R^{H4}, R^{H5}, R^{H6}, R^{J1}, R^{J2}, R^{J3}, R^{J4}, R^{J5}, R^{J6} , R^{J7}, R^{Y1}, R^{Y2}, R^{Y3}, R^{Y4}, R^{Y5}, R^{Y6}, R^{Y7}, R^{U1}, R^{U2}, R^{U3}, R^{U4}, R^{U5}, R^{U6}, R^{U7}, R^{U8}, R^{K1}, R^{K2}, R^{K3}, R^{K4}, R^{K5}, R^{K6}, R^{K7} and R^{K8} are identical or different and are each independently selected from the group consisting of a hydrogen atom, halogen and C₁₋₆ alkyl; Rⁿ², Rⁿ³, Rⁿ⁶ and R^{H03} are identical or different and are each independently a hydrogen atom or C₁₋₆ alkyl; and R^{9a} is selected from the group consisting of C₁₋₆ alkyl, C₁₋₆ hydroxyalkyl, 3- to 6-membered cycloalkyl and 3- to 6-membered heterocyclyl;
more preferably, R^{H0}, R^{J0} and R^{Y0} are identical or different and are each independently selected from the group consisting of a hydrogen atom, -NRⁿ²Rⁿ³ and -NRⁿ⁶C(O)R^{9a}; R^{H1} R^{H2}, R^{H3}, R^{H4}, R^{H5}, R^{H6}, R^{J1}, R^{J2}, R^{J3}, R^{J4}, R^{J5}, R^{J6} , R^{J7}, R^{Y1}, R^{Y2}, R^{Y3}, R^{Y4}, R^{Y5}, R^{Y6}, R^{Y7}, R^{U1}, R^{U2}, R^{U3}, R^{U4}, R^{U5}, R^{U6}, R^{U7}, R^{U8}, R^{K1}, R^{K2}, R^{K3}, R^{K4}, R^{K5}, R^{K6}, R^{K7} and R^{K8} are identical or different and are each independently selected from the group consisting of a hydrogen atom, halogen and C₁₋₆ alkyl; Rⁿ², Rⁿ³, Rⁿ⁶ and R^{H03} are identical or different and are each independently a hydrogen atom or C₁₋₆ alkyl; and R^{9a} is C₁₋₆ alkyl or 3- to 6-membered cycloalkyl;
and most preferably, R^{H0}, R^{J0} and R^{Y0} are all -NH₂; R^{H03} is a hydrogen atom or C₁₋₆ alkyl; R^{H1}, R^{H2}, R^{H3}, R^{H4}, R^{H5}, R^{H6}, R^{J1}, R^{J2}, R^{J3}, R^{J4}, R^{J5}, R^{J6}, R^{J7}, R^{Y1}, R^{Y2}, R^{Y3}, R^{Y4}, R^{Y5}, R^{Y6}, R^{Y7}, R^{U1}, R^{U2}, R^{U3}, R^{U4}, R^{U5}, R^{U6}, R^{U7}, R^{U8}, R^{K1}, R^{K2}, R^{K3}, R^{K4}, R^{K5}, R^{K6}, R^{K7} and RK⁸ are all hydrogen atoms.

In some embodiments of the present disclosure, provided is the compound of general formula (I), general formula (II), general formula (III) or general formula (IV) or the pharmaceutically acceptable salt thereof, wherein 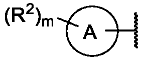 is selected from the group consisting of 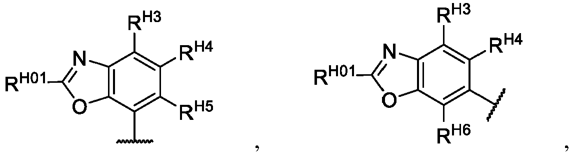 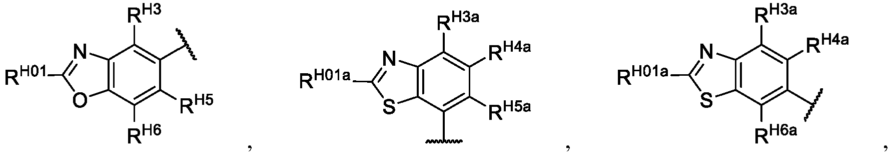 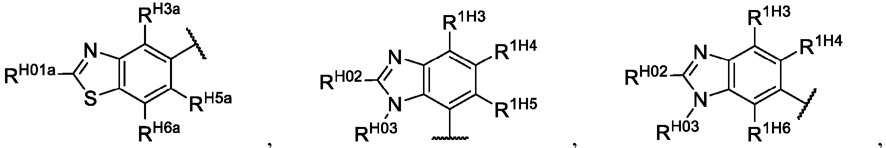 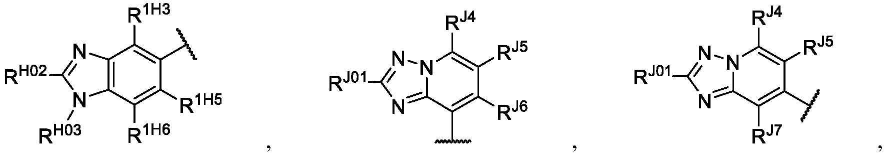 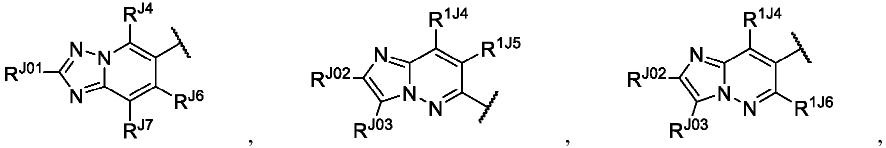 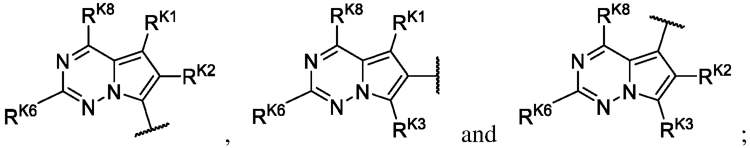 R^{H01}, R^{H01a}, R^{H02}, R^{H03} ,R^{J01}, R^{J02}, R^{J03}, R^{H3}, R^{H4}, R^{H5}, R^{H6}, R^{H3a}, R^{H4a}, R^{H5a}, R^{H6a}, R^{1H3}, R^{1H4}, R^{1H5}, R^{1H6}, R^{J4}, R^{J5}, R^{J6} , R^{J7}, R^{1J4}, R^{1J5}, R^{1J6}, R^{K1}, R^{K2}, R^{K3}, R^{K6} and R^{K8} are identical or different and are each independently selected from the group consisting of a hydrogen atom, halogen, alkyl, alkenyl, alkynyl, nitro, cyano, -NRⁿ²Rⁿ³, -C(O)NRⁿ⁴Rⁿ⁵, -NRⁿ⁶C(O)R^{9a}, - C(O)R^{9b}, -OC(O)R^{9c}, -C(O)OR^{10a}, -OR^{10b}, -S(O)ₜR^{10c}, cycloalkyl, heterocyclyl, aryl and heteroaryl; wherein the alkyl, alkenyl, alkynyl, alkoxy, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl; t, Rⁿ², Rⁿ³, Rⁿ⁴, Rⁿ⁵ Rⁿ⁶, Rⁿ⁷, Rⁿ⁸, R^{9a}, R^{9b}, R^{9c}, R^{10a}, R^{10b} and R^{10c} are as defined in general formula (I);
preferably, 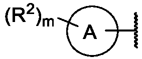 is selected from the group consisting of 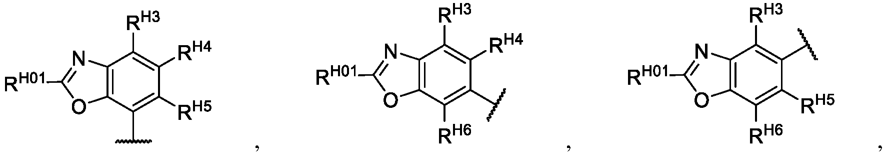 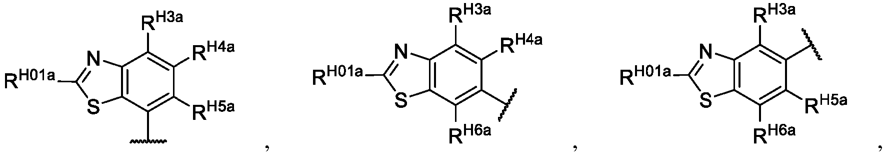 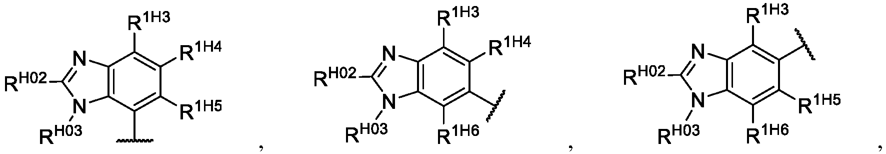 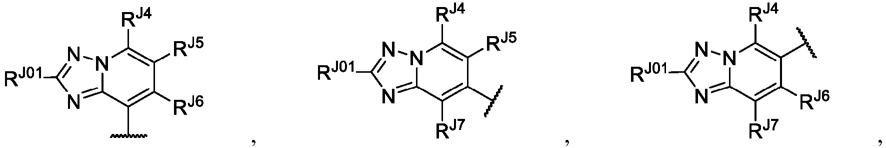 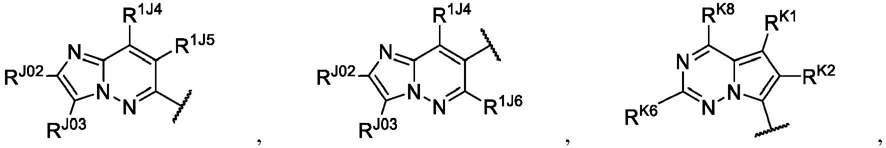 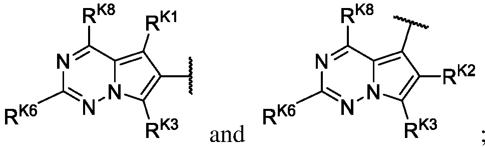 R^{H01}, R^{H01a}, R^{H02}, R^{H03}, R^{J01}, R^{J02} and R^{J03} are identical or different and are each independently selected from the group consisting of a hydrogen atom, C₁₋₆ alkyl, C₁₋₆ alkoxy, C₁₋₆ haloalkyl, C₂₋₆ alkenyl, C₂₋₆ alkynyl, C₁₋₆ haloalkoxy, -NRⁿ²Rⁿ³ and -NRⁿ⁶C(O)R^{9a}, wherein Rⁿ², Rⁿ³ and Rⁿ⁶ are identical or different and are each independently a hydrogen atom or C₁₋₆ alkyl, and R^{9a} is C₁₋₆ alkyl or 3- to 6-membered cycloalkyl; R^{H3}, R^{H4}, R^{H5}, R^{H6} R^{H3a}, R^{H4a}, R^{H5a}, R^{H6a}, R^{1H3}, R^{1H4}, R^{1H5}, R^{1H6}, R^{J4}, R^{J5}, R^{J6}, R^{J7}, R^{1J4}, R^{1J5}, R^{1J6}, R^{K1}, R^{K2}, R^{K3}, R^{K6} and R^{K8} are identical or different and are each independently selected from the group consisting of a hydrogen atom, halogen, C₁₋₆ alkyl, C₁₋₆ alkoxy, C₁₋₆ haloalkyl and C₁₋₆ haloalkoxy;
more preferably, 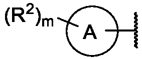 is 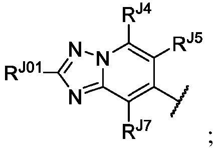 R^{J01} is selected from the group consisting of a hydrogen atom, C₁₋₆ alkyl, -NRⁿ²Rⁿ³ and -NRⁿ⁶C(O)R^{9a}, wherein Rⁿ², Rⁿ³ and Rⁿ⁶ are identical or different and are each independently a hydrogen atom or C₁₋₆ alkyl, and R^{9a} is selected from the group consisting of C₁₋₆ alkyl, C₁₋₆ hydroxyalkyl, 3- to 6-membered cycloalkyl and 3- to 6-membered heterocyclyl; R^{J4}, R^{J5} and R^{J7} are identical or different and are each independently selected from the group consisting of a hydrogen atom, halogen, C₁₋₆ alkyl, C₁₋₆ alkoxy, C₁₋₆ haloalkyl and C₁₋₆ haloalkoxy; further preferably, 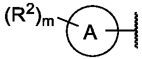 is 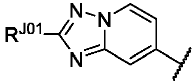 ; R^{J01} is -NRⁿ²Rⁿ³ or - NRⁿ⁶C(O)R^{9a}, wherein Rⁿ², Rⁿ³ and Rⁿ⁶ are identical or different and are each independently a hydrogen atom or C₁₋₆ alkyl, and R^{9a} is C₁₋₆ alkyl or 3- to 6-membered cycloalkyl; still further preferably, 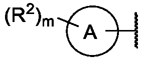 is 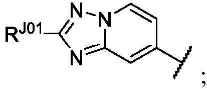 R^{J01} is -NRⁿ²Rⁿ³ or - NRⁿ⁶C(O)R^{9a}, wherein Rⁿ², Rⁿ³ and Rⁿ⁶ are identical or different and are each independently a hydrogen atom or C₁₋₆ alkyl, and R^{9a} is C₁₋₆ alkyl; yet still further preferably, 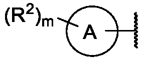 is selected from the group consisting of 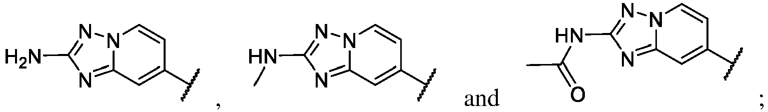 and most preferably,. 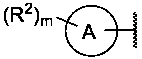 is

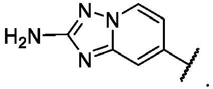

In some embodiments of the present disclosure, provided is the compound of general formula (I), general formula (II), general formula (III) or general formula (IV) or the pharmaceutically acceptable salt thereof, wherein 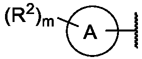 is 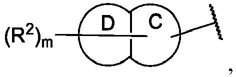 wherein ring C and ring D are identical or different and are each independently selected from the group consisting of 5- or 6-membered cycloalkyl, 5- or 6-membered heteroaryl and 5- or 6-membered heterocyclyl, and R² and m are as defined in general formula (I); preferably, 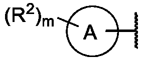 is 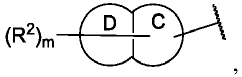 wherein ring D is 5- or 6-membered heteroaryl, ring C is 5- or 6-membered heterocyclyl, and R² and m are as defined in general formula (I); and more preferably, 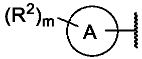 is

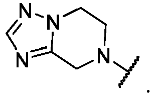

In some embodiments of the present disclosure, provided is the compound of general formula (I), general formula (II), general formula (III), general formula (IV) or general formula (V) or the pharmaceutically acceptable salt thereof, wherein Z is selected from the group consisting of -NRⁿ¹-, -CR^{z1}R^{z2}- and -O-, wherein Rⁿ¹ , R^{z1} and R^{z2} are as defined in general formula (I); preferably, Z is selected from the group consisting of -NRⁿ¹-, - CR^{z1}R^{z2}- and -O-, wherein Rⁿ¹ is a hydrogen atom or C₁₋₆ alkyl, and R^{z1} and R^{z2} are identical or different and are each independently selected from the group consisting of a hydrogen atom, halogen and C₁₋₆ alkyl; more preferably, Z is -CR^{z1}R^{z2}- or -O-, wherein R^{z1} and R^{z2} are identical or different and are each independently a hydrogen atom or C₁₋₆ alkyl; and most preferably, Z is -CH₂- or -O-.

In some embodiments of the present disclosure, provided is the compound of general formula (I), general formula (II), general formula (III), general formula (IV) or general formula (V) or the pharmaceutically acceptable salt thereof, wherein each R^{a} is identical or different and is independently halogen or C₁₋₆ alkyl; and v is 0, 1 or 2; preferably, each R^{a} is identical or different and is independently halogen or C₁₋₆ alkyl; and v is 0 or 1; more preferably, each R^{a} is identical or different and is independently halogen; and v is 0 or 1; and most preferably, v is 0.

In some embodiments of the present disclosure, provided is the compound of general formula (I), general formula (II), general formula (III), general formula (IV) or general formula (V) or the pharmaceutically acceptable salt thereof, wherein Rⁿ¹, Rⁿ², Rⁿ³, Rⁿ⁴, Rⁿ⁵, Rⁿ⁶, Rⁿ⁷ and Rⁿ⁸ are identical or different at each occurrence and are each independently selected from the group consisting of a hydrogen atom, C₁₋₆ alkyl, C₁₋₆ haloalkyl, C₁₋₆ hydroxyalkyl, 3- to 6-membered cycloalkyl and 3- to 6-membered heterocyclyl; preferably, Rⁿ¹, Rⁿ², Rⁿ³, Rⁿ⁴, Rⁿ⁵, Rⁿ⁶, Rⁿ⁷ and Rⁿ⁸ are identical or different at each occurrence and are each independently a hydrogen atom or C₁₋₆ alkyl.

In some embodiments of the present disclosure, provided is the compound of general formula (I), general formula (II), general formula (III), general formula (IV) or general formula (V) or the pharmaceutically acceptable salt thereof, wherein R^{z1} and R^{z2} are identical or different and are each independently selected from the group consisting of a hydrogen atom, halogen and C₁₋₆ alkyl; preferably, R^{z1} and R^{z2} are identical or different and are each independently a hydrogen atom or C₁₋₆ alkyl; and more preferably, R^{z1} and R^{z2} are both hydrogen atoms.

In some embodiments of the present disclosure, provided is the compound of general formula (I), general formula (II), general formula (III), general formula (IV) or general formula (V) or the pharmaceutically acceptable salt thereof, wherein R^{9a}, R^{9b}, R^{9c}, R^{10a}, R^{10b} and R^{10c} are identical or different at each occurrence and are each independently selected from the group consisting of a hydrogen atom, C₁₋₆ alkyl, C₁₋₆ hydroxyalkyl, 3-to 6-membered cycloalkyl, 3- to 6-membered heterocyclyl, 6- to 10-membered aryl and 5- to 10-membered heteroaryl, wherein the C₁₋₆ alkyl, 3- to 6-membered cycloalkyl, 3- to 6-membered heterocyclyl, 6- to 10-membered aryl and 5- to 10-membered heteroaryl are each independently optionally substituted with one or more substituents selected from the group consisting of halogen, oxo, C₁₋₆ alkyl, C₁₋₆ alkoxy, C₁₋₆ haloalkyl and C₁₋₆ haloalkoxy; preferably, R^{9a}, R^{9b}, R^{9c}, R^{10a}, R^{10b} and R^{10c} are identical or different at each occurrence and are each independently selected from the group consisting of a hydrogen atom, C₁₋₆ alkyl and C₁₋₆ hydroxyalkyl, wherein the C₁₋₆ alkyl is optionally substituted with one or more halogens; and more preferably, R^{9a}, R^{9b}, R^{9c}, R^{10a}, R^{10b} and R^{10c} are identical or different at each occurrence and are each independently a hydrogen atom or C₁₋₆ alkyl, wherein the C₁₋₆ alkyl is optionally substituted with one or more halogens.

In some embodiments of the present disclosure, provided is the compound of general formula (II) or the pharmaceutically acceptable salt thereof, wherein ring A is selected from the group consisting of pyridinyl, benzimidazolyl, benzoxazolyl, benzothiazolyl, pyrrolotriazinyl, 5,6,7,8-tetrahydro-triazolopyrazinyl, imidazopyridazinyl and [1,2,4]triazolo[1,5-a]pyridinyl; n is 2 or 3; and each R¹ is identical or different and is independently selected from the group consisting of halogen, C₁₋₆ alkyl, C₁₋₆ haloalkyl and C₁₋₆ haloalkoxy; Z is -CR^{z1}R^{z2}- and -O-, wherein R^{z1} and R^{z2} are identical or different and are each independently a hydrogen atom or C₁₋₆ alkyl; each R² is identical or different and is each independently -NRⁿ²Rⁿ³- or -NRⁿ⁶C(O)R^{9a}, wherein Rⁿ², Rⁿ³ and Rⁿ⁶ are identical or different and are each independently a hydrogen atom or C₁₋₆ alkyl, and R^{9a} is C₁₋₆ alkyl or 3- to 6-membered cycloalkyl, and m is 0, 1 or 2; each R^{a} is identical or different and is each independently halogen or C₁₋₆ alkyl, and v is 0, 1 or 2; R³ and R⁴ are identical or different and are each independently a hydrogen atom or C₁₋₆ alkyl, or R³ and R⁴, together with the carbon atom to which they are attached, form 3- to 6-membered cycloalkyl; R⁵ and R⁶ are identical or different and are each independently a hydrogen atom or C₁₋₆ alkyl; and R⁷ and R⁸ are identical or different and are each independently a hydrogen atom or C₁₋₆ alkyl.

In some embodiments of the present disclosure, provided is the compound of general formula (IV) or the pharmaceutically acceptable salt thereof, wherein ring A is [1,2,4]triazolo[1,5-a]pyridinyl; R^{1a} and R^{1b} are identical or different and are each independently halogen or C₁₋₆ haloalkoxy; v is 0; Z is -CR²¹R²²- and -O-, wherein R^{z1} and R^{z2} are identical or different and are each independently a hydrogen atom or C₁₋₆ alkyl; R² is -NRⁿ²Rⁿ³, wherein Rⁿ² and Rⁿ³ are identical or different and are each independently a hydrogen atom or C₁₋₆ alkyl, and m is 1.

**Table A. Typical compounds of the present disclosure include, but are not limited to:**

| Example No. | Structures and names of compounds |
|---|---|
| 1 | 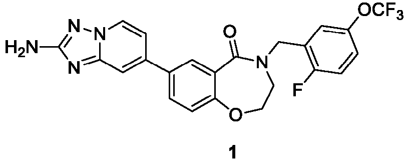 |
| | 7-(2-Amino-[1,2,4]triazolo[1,5-*a*]pyridin-7-yl)-4-(2-fluoro-5-(trifluoromethoxy)benzyl)-3,4-dihydrobenzo[*f*][1,4]oxazepin-5(2*H*)-one 1 |
| 2 | 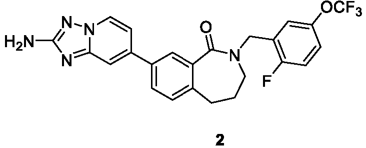 |
| | 8-(2-Amino-[1,2,4]triazolo[1,5-*a*]pyridin-7-yl)-2-(2-fluoro-5-(trifluoromethoxy)benzyl)-2,3,4,5-tetrahydro-1*H*-benzo [c] azepin-1-one 2 |

Another aspect of the present disclosure relates to a compound of general formula (IA) or a salt thereof: 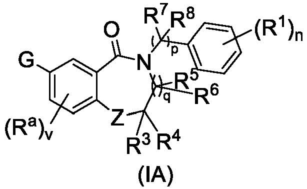 wherein:
G is 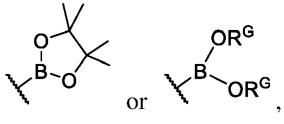 and R^{G} is a hydrogen atom or C₁₋₆ alkyl;
R¹, R³ to R⁸, R^{a}, v, Z, p, q and n are as defined in general formula (I).

Another aspect of the present disclosure relates to a compound of general formula (IIA) or a salt thereof: wherein: 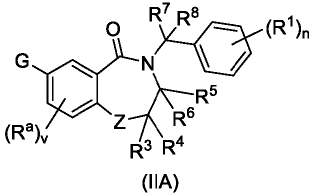
G is 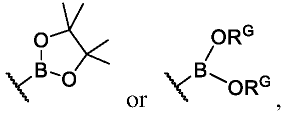 and R^{G} is a hydrogen atom or C₁₋₆ alkyl;
R¹, R³ to R⁸, R^{a}, v, Z and n are as defined in general formula (II).

Another aspect of the present disclosure relates to a compound of general formula (IIIA) or a salt thereof: wherein: 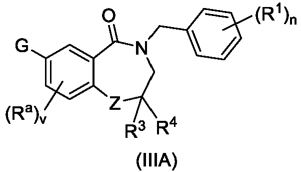
G is 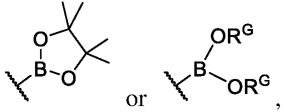 and R^{G} is a hydrogen atom or C₁₋₆ alkyl;
R¹, R³, R⁴, R^{a}, v, Z and n are as defined in general formula (III).

Another aspect of the present disclosure relates to a compound of general formula (IVA) or a salt thereof: 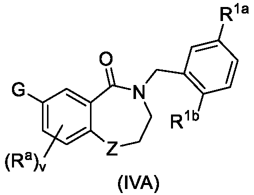 wherein:
G is 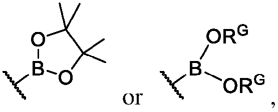 and R^{G} is a hydrogen atom or C₁₋₆ alkyl;
R^{1a}, R^{1b}, R^{a}, v and Z are as defined in general formula (IV).

**Table B. Typical intermediate compounds of the present disclosure include, but are not limited to:**

| Example No. | Structures and names of compounds |
|---|---|
| 1g | 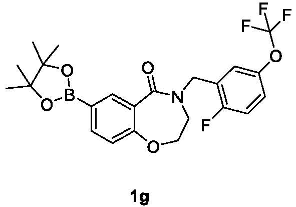 |
| | 4-(2-Fluoro-5-(trifluoromethoxy)benzyl)-7-(4,4,5,5-tetramethyl- |
| | 1,3,2-dioxaborolan-2-yl)-3,4-dihydrobenzo[*f*][1,4]oxazepin-5(2*H*)-one **1g** |
| 2e | 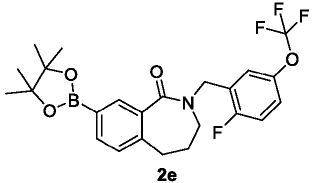 |
| | 2-(2-Fluoro-5-(trifluoromethoxy)benzyl)-8-(4,4,5,5-tetramethyl-1,3,2-dioxaborolan-2-yl)-2,3,4,5-tetrahydro-1*H*-benzo [c] azepan-1-one **2e** |

Another aspect of the present disclosure relates to a method for preparing a compound of general formula (I) or a pharmaceutically acceptable salt thereof, the method comprising the following step: 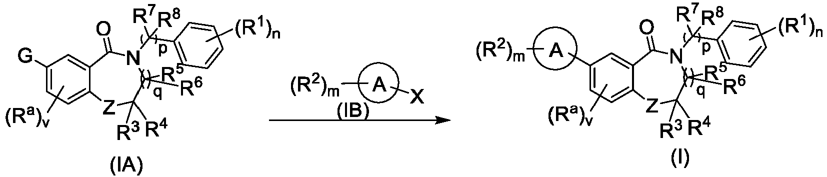 conducting a coupling reaction of a compound of general formula (IA) or a salt thereof with a compound of general formula (IB) or a salt thereof to give the compound of general formula (I) or the pharmaceutically acceptable salt thereof,
wherein:
G is 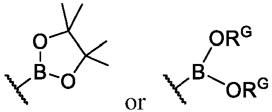 , and R^{G} is a hydrogen atom or C₁₋₆ alkyl;
X is halogen, preferably Br;
ring A, R¹ to R⁸, R^{a}, v, Z, p, q, m and n are as defined in general formula (I).

Another aspect of the present disclosure relates to a method for preparing a compound of general formula (II) or a pharmaceutically acceptable salt thereof, the method comprising the following step: 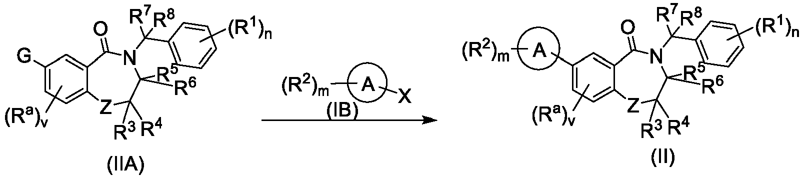 conducting a coupling reaction of a compound of general formula (IIA) or a salt thereof with a compound of general formula (IB) or a salt thereof to give the compound of general formula (II) or the pharmaceutically acceptable salt thereof,
wherein:
G is 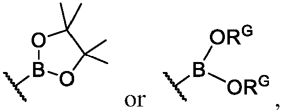 and R^{G} is a hydrogen atom or C₁₋₆ alkyl;
X is halogen, preferably Br;
ring A, R¹ to R⁸, R^{a}, v, Z, m and n are as defined in general formula (II).

Another aspect of the present disclosure relates to a method for preparing a compound of general formula (III) or a salt thereof, the method comprising the following step: 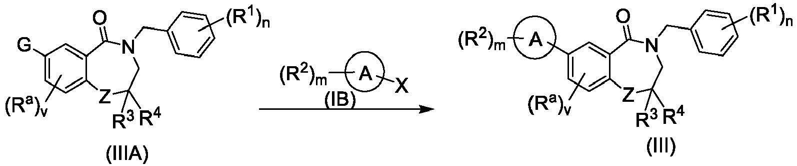 conducting a coupling reaction of a compound of general formula (IIIA) or a salt thereof with a compound of general formula (IB) or a salt thereof to give the compound of general formula (III) or the pharmaceutically acceptable salt thereof,
wherein:
G is 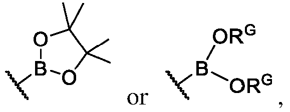 and R^{G} is a hydrogen atom or C₁₋₆ alkyl;
X is halogen, preferably Br;
ring A, R¹ to R⁴, R^{a}, v, Z, m and n are as defined in general formula (III).

Another aspect of the present disclosure relates to a method for preparing a compound of general formula (IV) or a pharmaceutically acceptable salt thereof, the method comprising the following step: 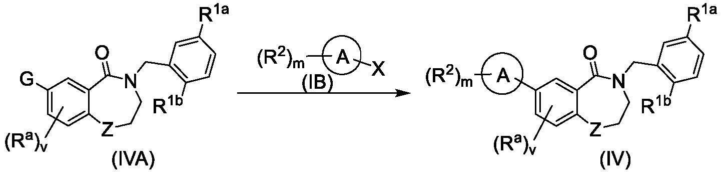 conducting a coupling reaction of a compound of general formula (IVA) or a salt thereof with a compound of general formula (IB) or a salt thereof to give the compound of general formula (IV) or the pharmaceutically acceptable salt thereof, wherein:
G is 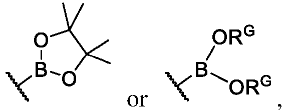 and R^{G} is a hydrogen atom or C₁₋₆ alkyl;
X is halogen, preferably Br;
ring A, R^{1a}, R^{1b}, R^{a}, v, R², m and Z are as defined in general formula (IV).

Another aspect of the present disclosure relates to a method for preparing a compound of general formula (V) or a pharmaceutically acceptable salt thereof, the method comprising the following step: 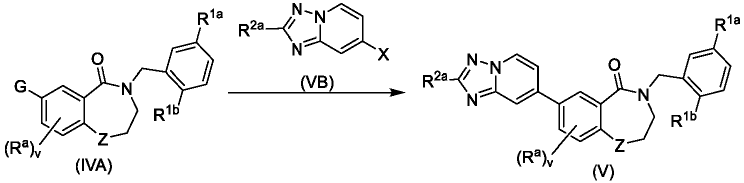 conducting a coupling reaction of a compound of general formula (IVA) or a salt thereof with a compound of general formula (VB) or a salt thereof to give the compound of general formula (V) or the pharmaceutically acceptable salt thereof, wherein:
G is 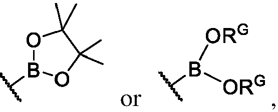 and R^{G} is a hydrogen atom or C₁₋₆ alkyl;
X is halogen, preferably Br;
R^{1a}, R^{1b}, R^{a}, v, R^{2a} and Z are as defined in general formula (V).

Another aspect of the present disclosure relates to a pharmaceutical composition comprising the compound of general formula (I), general formula (II), general formula (III), general formula (IV) or general formula (V) of the present disclosure or the compound shown in Table A or the pharmaceutically acceptable salt thereof, and one or more pharmaceutically acceptable carriers, diluents or excipients.

The present disclosure further relates to use of the compound of general formula (I), general formula (II), general formula (III), general formula (IV) or general formula (V) or the compound shown in Table A or the pharmaceutically acceptable salt thereof, or the pharmaceutical composition comprising same in the preparation of a medicament for inhibiting RIPK1 kinase.

The present disclosure further relates to use of the compound of general formula (I), general formula (II), general formula (III), general formula (IV) or general formula (V) or the compound shown in Table A or the pharmaceutically acceptable salt thereof, or the pharmaceutical composition comprising same in the preparation of a medicament for treating and/or preventing a RIPK1 kinase-mediated disease or disorder.

The present disclosure further relates to use of the compound of general formula (I), general formula (II), general formula (III), general formula (IV) or general formula (V) or the compound shown in Table A or the pharmaceutically acceptable salt thereof, or the pharmaceutical composition comprising same in the preparation of a medicament for treating and/or preventing a RIPK1 kinase-mediated disease or disorder, wherein the disease or disorder is selected from the group consisting of a CNS disease, an autoimmune disease, an inflammatory disease, an ischemic disease and a cancer; preferably, the CNS disease is a neurodegenerative disease.

The present disclosure further relates to use of the compound of general formula (I), general formula (II), general formula (III), general formula (IV) or general formula (V) or the compound shown in Table A or the pharmaceutically acceptable salt thereof, or the pharmaceutical composition comprising same in the preparation of a medicament for treating and/or preventing amyotrophic lateral sclerosis, multiple sclerosis, Alzheimer's disease, Huntington's chorea, Friedreich's ataxia, Parkinson's disease, spinal muscular atrophy, stroke, human immunodeficiency virus-associated dementia, autism, schizophrenia, rheumatoid arthritis, osteoarthritis, ankylosing spondylitis, systemic onset juvenile idiopathic arthritis, psoriasis, dermatitis, systemic lupus erythematosus, systemic inflammatory response syndrome, pancreatitis, encephalitis, nonalcoholic steatohepatitis, alcoholic steatohepatitis, autoimmune hepatitis, autoimmune hepatobiliary diseases, primary sclerosing cholangitis, nephritis, ulcerative colitis, Crohn's disease, retinal degenerative diseases, retinal detachment, retinitis pigmentosa, macular degeneration, pancreatitis, Sjögren's syndrome, systemic scleroderma, ischemia-reperfusion injury of solid organs, cerebral ischemia, ischemic heart disease, acute kidney injury, ischemic brain injury, sepsis, diabetes and atherosclerosis; preferably, in the preparation of a medicament for treating and/or preventing amyotrophic lateral sclerosis, multiple sclerosis, Alzheimer's disease, Huntington's chorea, Friedreich's ataxia, Parkinson's disease and spinal muscular atrophy.

The present disclosure further relates to a method for inhibiting RIPK1 kinase, which comprises administering to a patient in need thereof a therapeutically effective amount of the compound of general formula (I), general formula (II), general formula (III), general formula (IV) or general formula (V) or the compound shown in Table A or the pharmaceutically acceptable salt thereof, or the pharmaceutical composition comprising same.

The present disclosure further relates to a method for treating and/or preventing a RIPK1 kinase-mediated disease or disorder, which comprises administering to a patient in need thereof a therapeutically effective amount of the compound of general formula (I), general formula (II), general formula (III), general formula (IV) or general formula (V) or the compound shown in Table A or the pharmaceutically acceptable salt thereof, or the pharmaceutical composition comprising same.

The present disclosure further relates to a method for treating and/or preventing a RIPK1 kinase-mediated disease or disorder, which comprises administering to a patient in need thereof a therapeutically effective amount of the compound of general formula (I), general formula (II), general formula (III), general formula (IV) or general formula (V) or the compound shown in Table A or the pharmaceutically acceptable salt thereof, or the pharmaceutical composition comprising same, wherein the disease or disorder is selected from the group consisting of a CNS disease, an autoimmune disease, an inflammatory disease, an ischemic disease and a cancer; preferably, the CNS disease is a neurodegenerative disease.

The present disclosure further relates to a method for treating and/or preventing amyotrophic lateral sclerosis, multiple sclerosis, Alzheimer's disease, Huntington's chorea, Friedreich's ataxia, Parkinson's disease, spinal muscular atrophy, stroke, human immunodeficiency virus-associated dementia, autism, schizophrenia, rheumatoid arthritis, osteoarthritis, ankylosing spondylitis, systemic onset juvenile idiopathic arthritis, psoriasis, dermatitis, systemic lupus erythematosus, systemic inflammatory response syndrome, pancreatitis, encephalitis, nonalcoholic steatohepatitis, alcoholic steatohepatitis, autoimmune hepatitis, autoimmune hepatobiliary diseases, primary sclerosing cholangitis, nephritis, ulcerative colitis, Crohn's disease, retinal degenerative diseases, retinal detachment, retinitis pigmentosa, macular degeneration, pancreatitis, Sjögren's syndrome, systemic scleroderma, ischemia-reperfusion injury of solid organs, cerebral ischemia, ischemic heart disease, acute kidney injury, ischemic brain injury, sepsis, diabetes and atherosclerosis, preferably a method for treating and/or preventing amyotrophic lateral sclerosis, multiple sclerosis, Alzheimer's disease, Huntington's chorea, Friedreich's ataxia, Parkinson's disease and spinal muscular atrophy, which comprises administering to a patient in need thereof a therapeutically effective amount of the compound of general formula (I), general formula (II), general formula (III), general formula (IV) or general formula (V) or the compound shown in Table A or the pharmaceutically acceptable salt thereof, or the pharmaceutical composition comprising same.

The present disclosure further relates to a compound of general formula (I), general formula (II), general formula (III), general formula (IV) or general formula (V) or a compound shown in Table A or a pharmaceutically acceptable salt thereof, or a pharmaceutical composition comprising same, for use as a medicament.

The present disclosure further relates to a compound of general formula (I), general formula (II), general formula (III), general formula (IV) or general formula (V) or a compound shown in Table A or a pharmaceutically acceptable salt thereof, or a pharmaceutical composition comprising same, for use as a RIPK1 kinase inhibitor.

The present disclosure further relates to a compound of general formula (I), general formula (II), general formula (III), general formula (IV) or general formula (V) or a compound shown in Table A or a pharmaceutically acceptable salt thereof, or a pharmaceutical composition comprising same, for use in the treatment and/or prevention of a RIPK1 kinase-mediated disease or disorder.

The present disclosure further relates to a compound of general formula (I), general formula (II), general formula (III), general formula (IV) or general formula (V) or a compound shown in Table A or a pharmaceutically acceptable salt thereof, or a pharmaceutical composition comprising same, for use in the treatment and/or prevention of a RIPK1 kinase-mediated disease or disorder, wherein the disease or disorder is selected from the group consisting of a CNS disease, an autoimmune disease, an inflammatory disease, an ischemic disease and a cancer; preferably, the CNS disease is a neurodegenerative disease.

The present disclosure further relates to a compound of general formula (I), general formula (II), general formula (III), general formula (IV) or general formula (V) or a compound shown in Table A or a pharmaceutically acceptable salt thereof, or a pharmaceutical composition comprising same, for use in the treatment and/or prevention of amyotrophic lateral sclerosis, multiple sclerosis, Alzheimer's disease, Huntington's chorea, Friedreich's ataxia, Parkinson's disease, spinal muscular atrophy, stroke, human immunodeficiency virus-associated dementia, autism, schizophrenia, rheumatoid arthritis, osteoarthritis, ankylosing spondylitis, systemic onset juvenile idiopathic arthritis, psoriasis, dermatitis, systemic lupus erythematosus, systemic inflammatory response syndrome, pancreatitis, encephalitis, nonalcoholic steatohepatitis, alcoholic steatohepatitis, autoimmune hepatitis, autoimmune hepatobiliary diseases, primary sclerosing cholangitis, nephritis, ulcerative colitis, Crohn's disease, retinal degenerative diseases, retinal detachment, retinitis pigmentosa, macular degeneration, pancreatitis, Sjögren's syndrome, systemic scleroderma, ischemia-reperfusion injury of solid organs, cerebral ischemia, ischemic heart disease, acute kidney injury, ischemic brain injury, sepsis, diabetes and atherosclerosis; preferably, for use in the treatment and/or prevention of amyotrophic lateral sclerosis, multiple sclerosis, Alzheimer's disease, Huntington's chorea, Friedreich's ataxia, Parkinson's disease and spinal muscular atrophy.

The neurodegenerative disease described herein may affect many activities of the body, such as balance, movement, speech, breathing, and cardiac function. The neurodegenerative disease may be a genetic disease or may be caused by a medical disease, such as alcoholism, tumors, stroke, toxins, chemical substances and viruses. Non-limiting examples of the neurodegenerative disease include Alzheimer's disease, amyotrophic lateral sclerosis (ALS or Lou Gehrig's disease or gradual freezing disease), Friedreich's ataxia, Huntington's chorea, Lewy body disease, Parkinson's disease and spinal muscular atrophy.

Non-limiting examples of the central nervous system (CNS) disease or disorder described herein include brain injury, spinal cord injury, dementia, stroke, Alzheimer's disease, amyotrophic lateral sclerosis, Parkinson's disease, Huntington's chorea, multiple sclerosis, diabetic neuropathy, polyglutamine (polyQ) diseases, stroke, Fahr's disease, Menkes disease, Wilson's disease, cerebral ischemia and prion diseases.

Preferably, the central nervous system (CNS) disease or disorder described herein includes motor neuron diseases (MNDs), and the motor neuron diseases refer to a group of diseases in which motor neurons of the human body are injured and gradually progress, mainly including amyotrophic lateral sclerosis, progressive bulbar palsy, primary lateral sclerosis and progressive muscular atrophy.

Preferably, the ischemic disease described herein refers to a disease characterized by a hypoxic state usually due to the obstruction of the arterial blood supply or inadequate blood flow leading to hypoxia in the tissue; organs in which the disease occurs include, but are not limited to, brain, heart, kidney and liver. Non-limiting examples of the ischemic disease include ischemic brain injury, cerebral ischemia, retinal ischemia/reperfusion injury and ischemia-reperfusion injury of solid organs (e.g., kidney).

Preferably, the cancer described herein is selected from the group consisting of leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous carcinoma, papillary carcinoma, cystadenocarcinoma, medullary carcinoma, bronchogenic carcinoma, liver cancer, cholangiocarcinoma, choriocarcinoma, seminoma, embryonal carcinoma, Wilms tumor, cervical cancer, endometrial cancer, testicular cancer, lung cancer, bladder cancer, neuroglioma, medulloblastoma, craniopharyngioma, ependymoma, pinealoma, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, small intestine cancer, gallbladder cancer, pediatric tumor, urothelial cancer, ureteral tumor, thyroid cancer, osteoma, neuroblastoma, brain tumor and myeloma; wherein, colorectal cancer is preferably colon cancer or rectal cancer; neuroglioma is preferably selected from the group consisting of astrocytoma, glioblastoma and oligodendroglioma.

The active compound may be formulated into a form suitable for administration by any suitable route, and one or more pharmaceutically acceptable carriers are used to formulate the composition of the present disclosure by conventional methods. Thus, the active compound of the present disclosure may be formulated into a variety of dosage forms for oral administration, administration by injection (e.g., intravenous, intramuscular, or subcutaneous), or administration by inhalation or insufflation. The compound of the present disclosure may also be formulated into a sustained-release dosage form, such as tablets, hard or soft capsules, aqueous or oily suspensions, emulsions, injections, dispersible powders or granules, suppositories, lozenges or syrups.

As a general guide, the active compound is preferably in the form of a unit dose, or in the form of a single dose that can be administered by the patient. The unit dose of the compound or composition of the present disclosure may be expressed in the form of a tablet, capsule, cachet, vial, powder, granule, lozenge, suppository, regenerating powder, or liquid formulation. A suitable unit dose may be 0.1-1000 mg.

The pharmaceutical composition of the present disclosure may comprise, in addition to the active compound, one or more auxiliary materials selected from the group consisting of a filler (diluent), a binder, a wetting agent, a disintegrant, an excipient, and the like. Depending on the method of administration, the composition may comprise 0.1 to 99 wt.% of the active compound.

The tablet comprises the active ingredient, and non-toxic pharmaceutically acceptable excipients that are used for mixing and are suitable for the preparation of the tablet. These excipients may be an inert excipient, a granulating agent, a disintegrant, a binder, and a lubricant. These tablets may be uncoated or coated using known techniques that mask the taste of the drug or delay disintegration and absorption in the gastrointestinal tract, thereby providing a sustained-release effect over an extended period of time.

An oral formulation may also be provided in the form of a soft gelatin capsule in which the active ingredient is mixed with an inert solid diluent or with a water-soluble carrier or oil vehicle.

An aqueous suspension comprises the active substance and an excipient that is used for mixing and is suitable for the preparation of the aqueous suspension. Such an excipient is a suspending agent, a dispersant, or a wetting agent. The aqueous suspension may also comprise one or more preservatives, one or more colorants, one or more corrigents, and one or more sweeteners.

An oil suspension may be formulated by suspending the active ingredient in a vegetable oil, or in a mineral oil. The oil suspension may comprise a thickening agent. The sweeteners and corrigents described above may be added to provide a palatable formulation. Antioxidants may also be added to preserve the compositions.

The pharmaceutical composition of the present disclosure may also be in the form of an oil-in-water emulsion. The oil phase may be a vegetable oil or a mineral oil, or a mixture thereof. Suitable emulsifiers may be naturally occurring phospholipids, and the emulsion may also comprise a sweetener, a corrigent, a preservative, and an antioxidant. Such a formulation may also comprise a palliative, a preservative, a colorant, and an antioxidant. The pharmaceutical composition of the present disclosure may be in the form of a sterile injectable aqueous solution. Acceptable vehicles or solvents that can be used include water, Ringer's solution, and isotonic sodium chloride solution. A sterile injectable formulation may be a sterile injectable oil-in-water microemulsion in which an active ingredient is dissolved in an oil phase. The injection or microemulsion can be locally injected into the bloodstream of a patient in large quantities. Alternatively, it may be desirable to administer the solution and microemulsion in such a way as to maintain a constant circulating concentration of the compound of the present disclosure. To maintain such a constant concentration, a continuous intravenous delivery device may be used. An example of such a device is a Deltec CADD-PLUS. TM. 5400 intravenous injection pump.

The pharmaceutical composition of the present disclosure may be in the form of a sterile injectable aqueous or oil suspension for intramuscular and subcutaneous administration. The suspension can be prepared according to the prior art using those suitable dispersants or wetting agents and suspending agents as described above. The sterile injectable formulation may also be a sterile injection or suspension prepared in a parenterally acceptable non-toxic diluent or solvent. In addition, a sterile fixed oil may be conventionally used as a solvent or a suspending medium. For this purpose, any blend fixed oil may be used. In addition, fatty acids may also be used to prepare injections. The compound of the present disclosure may be administered in the form of a suppository for rectal administration. Such a pharmaceutical composition can be prepared by mixing a drug with a suitable non-irritating excipient which is a solid at ambient temperature but a liquid in the rectum and therefore will melt in the rectum to release the drug.

The compound of the present disclosure can be administered in the form of dispersible powders and granules that are formulated into aqueous suspensions by adding water. Such a pharmaceutical composition can be prepared by mixing the active ingredient with a dispersant or a wetting agent, a suspending agent, or one or more preservatives.

As is well known to those skilled in the art, the dosage of the drug depends on a variety of factors, including, but not limited to, the activity of the particular compound used, the age of the patient, the body weight of the patient, the health condition of the patient, the behavior of the patient, the diet of the patient, the time of administration, the route of administration, the rate of excretion, the combination of drugs, the severity of the disease, and the like. In addition, the optimal treatment regimen, such as the mode of treatment, the daily dose of the compound, or the type of pharmaceutically acceptable salts, can be verified according to conventional treatment regimens.

### Terminology

Unless otherwise stated, the terms used in the specification and claims have the following meanings.

The term "alkyl" refers to a saturated straight-chain or branched-chain aliphatic hydrocarbon group having 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkyl). The alkyl is preferably an alkyl group having 1 to 12 carbon atoms (i.e., C₁₋₁₂ alkyl), and more preferably an alkyl group having 1 to 6 carbon atoms (i.e., C₁₋₆ alkyl). Non-limiting examples include: methyl, ethyl, n-propyl, isopropyl, n-butyl, isobutyl, tert-butyl, sec-butyl, n-pentyl, 1,1-dimethylpropyl, 1,2-dimethylpropyl, 2,2-dimethylpropyl, 1-ethylpropyl, 2-methylbutyl, 3-methylbutyl, n-hexyl, 1-ethyl-2-methylpropyl, 1,1,2-trimethylpropyl, 1,1-dimethylbutyl, 1,2-dimethylbutyl, 2,2-dimethylbutyl, 1,3-dimethylbutyl, 2-ethylbutyl, 2-methylpentyl, 3-methylpentyl, 4-methylpentyl, 2,3-dimethylbutyl, n-heptyl, 2-methylhexyl, 3-methylhexyl, 4-methylhexyl, 5-methylhexyl, 2,3-dimethylpentyl, 2,4-dimethylpentyl, 2,2-dimethylpentyl, 3,3-dimethylpentyl, 2-ethylpentyl, 3-ethylpentyl, n-octyl, 2,3-dimethylhexyl, 2,4-dimethylhexyl, 2,5-dimethylhexyl, 2,2-dimethylhexyl, 3,3-dimethylhexyl, 4,4-dimethylhexyl, 2-ethylhexyl, 3-ethylhexyl, 4-ethylhexyl, 2-methyl-2-ethylpentyl, 2-methyl-3-ethylpentyl, n-nonyl, 2-methyl-2-ethylhexyl, 2-methyl-3-ethylhexyl, 2,2-diethylpentyl, n-decyl, 3,3-diethylhexyl, 2,2-diethylhexyl, various branched-chain isomers thereof, and the like. Alkyl may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkylene" refers to a divalent alkyl group, wherein the alkyl is as defined above; it has 1 to 20 (e.g., 1, 2, 3, 4, 5, 6, 7, 8, 9, 10, 11, 12, 13, 14, 15, 16, 17, 18, 19, or 20) carbon atoms (i.e., C₁₋₂₀ alkylene). The alkylene is preferably an alkylene group having 1 to 12 carbon atoms (i.e., C₁₋₁₂ alkylene), and more preferably an alkylene group having 1 to 6 carbon atoms (i.e., C₁₋₆ alkylene). Non-limiting examples include: -CH₂-, -CH(CH₃)-, -C(CH₃)₂-, -CH₂CH₂-, -CH(CH₂CH₃)-, -CH₂CH(CH₃)-, -CH₂C(CH₃)₂-, -CH₂CH₂CH₂-, - CH₂CH₂CH₂CH₂-, and the like. Alkylene may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkenyl" refers to an alkyl group containing at least one carbon-carbon double bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkenyl). The alkenyl is preferably an alkenyl group having 2 to 6 carbon atoms (i.e., C₂₋₆ alkenyl). Non-limiting examples include: ethenyl, propenyl, isopropenyl, butenyl, and the like. Alkenyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkynyl" refers to an alkyl group containing at least one carbon-carbon triple bond in the molecule, wherein the alkyl group is as defined above, and it has 2 to 12 (e.g., 2, 3, 4, 5, 6, 7, 8, 9, 10, 11, or 12) carbon atoms (i.e., C₂₋₁₂ alkynyl). The alkynyl is preferably an alkynyl group having 2 to 6 carbon atoms (i.e., C₂₋₆ alkynyl). Non-limiting examples include: ethynyl, propynyl, butynyl, pentynyl, hexynyl, and the like. Alkynyl may be substituted or unsubstituted, and when it is substituted, it may be substituted at any accessible point of attachment, and the substituent is preferably selected from the group consisting of one or more of a D atom, alkoxy, halogen, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "alkoxy" refers to -O-(alkyl), wherein the alkyl is as defined above. Non-limiting examples include: methoxy, ethoxy, propoxy, butoxy, and the like. Alkoxy may be substituted or unsubstituted, and when it is substituted, it may be substituted at any accessible point of attachment, and the substituent is preferably selected from the group consisting of one or more of a D atom, halogen, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "cycloalkyl" refers to a saturated or partially unsaturated monocyclic all-carbon ring (i.e., monocyclic cycloalkyl) or polycyclic system (i.e., polycyclic cycloalkyl) having 3 to 20 (e.g., 3, 4, 5, 6, 7, 8, 9, 10, 11, 12, 13, 14, 15, 16, 17, 18, 19, or 20) ring atoms (i.e., 3- to 20-membered cycloalkyl). The cycloalkyl is preferably a cycloalkyl group having 3 to 12 ring atoms (i.e., 3- to 12-membered cycloalkyl), more preferably a cycloalkyl group having 3 to 8 ring atoms (i.e., 3- to 8-membered cycloalkyl), or preferably a cycloalkyl group having 3 to 6 ring atoms (i.e., 3- to 6-membered cycloalkyl).

Non-limiting examples of the monocyclic cycloalkyl include: cyclopropyl, cyclobutyl, cyclopentyl, cyclopentenyl, cyclohexyl, cyclohexenyl, cyclohexadienyl, cycloheptyl, cycloheptatrienyl, cyclooctyl, and the like.

The polycyclic cycloalkyl includes: spirocycloalkyl, fused cycloalkyl, and bridged cycloalkyl.

The term "spirocycloalkyl" refers to a polycyclic system in which a carbon atom (referred to as a spiro atom) is shared between rings, which may contain in the rings one or more double bonds or may contain in the rings one or more heteroatoms selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one all-carbon ring is contained and the point of attachment is on the all-carbon ring; and which has 5 to 20 (e.g., 5, 6, 7, 8, 9, 10, 11, 12, 13, 14, 15, 16, 17, 18, 19, or 20) ring atoms (i.e., 5- to 20-membered spirocycloalkyl). The spirocycloalkyl is preferably a spirocycloalkyl group having 6 to 14 ring atoms (i.e., 6- to 14-membered spirocycloalkyl), and more preferably a spirocycloalkyl group having 7 to 10 ring atoms (i.e., 7- to 10-membered spirocycloalkyl). The spirocycloalkyl includes monospirocycloalkyl and polyspirocycloalkyl (e.g., bispirocycloalkyl), preferably monospirocycloalkyl or bispirocycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospirocycloalkyl. Non-limiting examples include: 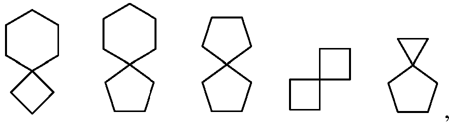 wherein the point of attachment may be at any position; 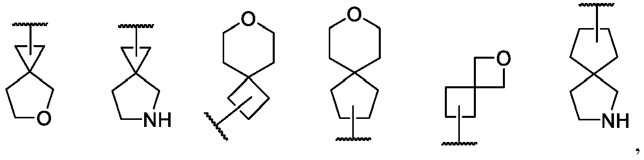 and the like.

The term "fused cycloalkyl" refers to a polycyclic system in which two adjacent carbon atoms are shared between rings, which is formed by fusing a monocyclic cycloalkyl group with one or more monocyclic cycloalkyl groups, or fusing a monocyclic cycloalkyl group with one or more of a heterocyclyl group, an aryl group, or a heteroaryl group, wherein the point of attachment is on a monocyclic cycloalkyl group; and which may contain in the rings one or more double bonds and has 5 to 20 (e.g., 5, 6, 7, 8, 9, 10, 11, 12, 13, 14, 15, 16, 17, 18, 19, or 20) ring atoms (i.e., 5- to 20-membered fused cycloalkyl). The fused cycloalkyl is preferably a fused cycloalkyl group having 6 to 14 ring atoms (i.e., 6- to 14-membered fused cycloalkyl), and more preferably a fused cycloalkyl group having 7 to 10 ring atoms (i.e., 7- to 10-membered fused cycloalkyl). The fused cycloalkyl includes bicyclic fused cycloalkyl and polycyclic fused cycloalkyl (e.g., tricyclic fused cycloalkyl and tetracyclic fused cycloalkyl), preferably bicyclic fused cycloalkyl or tricyclic fused cycloalkyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused cycloalkyl. Non-limiting examples include: 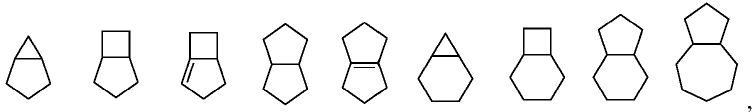 wherein the point of attachment may be at any position; 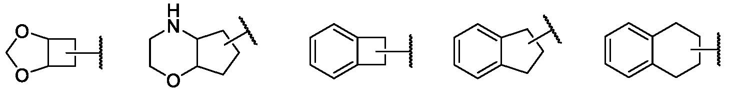 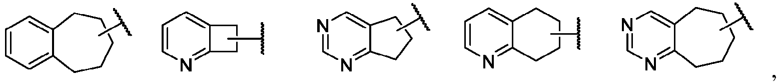 and the like.

The term "bridged cycloalkyl" refers to an all-carbon polycyclic system in which two carbon atoms that are not directly connected are shared between rings, which may contain in the rings one or more double bonds and has 5 to 20 (e.g., 5, 6, 7, 8, 9, 10, 11, 12, 13, 14, 15, 16, 17, 18, 19, or 20) carbon atoms (i.e., 5- to 20-membered bridged cycloalkyl). The bridged cycloalkyl is preferably a bridged cycloalkyl group having 6 to 14 carbon atoms (i.e., 6- to 14-membered bridged cycloalkyl), and more preferably a bridged cycloalkyl group having 7 to 10 carbon atoms (i.e., 7- to 10-membered bridged cycloalkyl). The bridged cycloalkyl includes bicyclic bridged cycloalkyl and polycyclic bridged cycloalkyl (e.g., tricyclic bridged cycloalkyl and tetracyclic bridged cycloalkyl), preferably bicyclic bridged cycloalkyl or tricyclic bridged cycloalkyl. Non-limiting examples include: 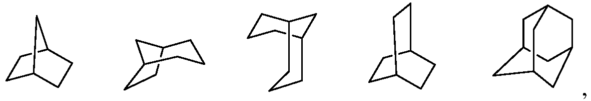 wherein the point of attachment may be at any position.

Cycloalkyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heterocyclyl" refers to a saturated or partially unsaturated monocyclic heterocyclic (i.e., monocyclic heterocyclyl) or polycyclic heterocyclic system (i.e., polycyclic heterocyclyl), which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 3 to 20 (e.g., 3, 4, 5, 6, 7, 8, 9, 10, 11, 12, 13, 14, 15, 16, 17, 18, 19, or 20) ring atoms (i.e., 3- to 20-membered heterocyclyl). The heterocyclyl is preferably a heterocyclyl group having 3 to 12 ring atoms (i.e., 3- to 12-membered heterocyclyl), further preferably a heterocyclyl group having 3 to 8 ring atoms (i.e., 3- to 8-membered heterocyclyl), more preferably a heterocyclyl group having 3 to 6 ring atoms (i.e., 3- to 6-membered heterocyclyl), or preferably a heterocyclyl group having 5 or 6 ring atoms (i.e., 5- or 6-membered heterocyclyl).

Non-limiting examples of the monocyclic heterocyclyl include: pyrrolidinyl, tetrahydropyranyl, 1,2,3,6-tetrahydropyridinyl, piperidinyl, piperazinyl, morpholinyl, thiomorpholinyl, homopiperazinyl, and the like.

The polycyclic heterocyclyl includes spiroheterocyclyl, fused heterocyclyl, and bridged heterocyclyl.

The term "spiroheterocyclyl" refers to a polycyclic heterocyclic system in which an atom (referred to as a spiro atom) is shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-), provided that at least one monocyclic heterocyclyl group is contained and the point of attachment is on the monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered spiroheterocyclyl). The spiroheterocyclyl is preferably a spiroheterocyclyl group having 6 to 14 ring atoms (i.e., 6- to 14-membered spiroheterocyclyl), more preferably a spiroheterocyclyl group having 7 to 10 ring atoms (i.e., 7- to 10-membered spiroheterocyclyl). The spiroheterocyclyl includes monospiroheterocyclyl and polyspiroheterocyclyl (e.g., bispiroheterocyclyl), preferably monospiroheterocyclyl or bispiroheterocyclyl, and more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered monospiroheterocyclyl. Non-limiting examples include: 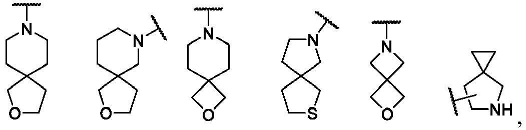 and the like.

The term "fused heterocyclyl" refers to a polycyclic heterocyclic system in which two adjacent atoms are shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); which is formed by fusing a monocyclic heterocyclyl group with one or more monocyclic heterocyclyl groups, or fusing a monocyclic heterocyclyl group with one or more of a cycloalkyl group, an aryl group, or a heteroaryl group, wherein the point of attachment is on a monocyclic heterocyclyl group; and which has 5 to 20 (e.g., 5, 6, 7, 8, 9, 10, 11, 12, 13, 14, 15, 16, 17, 18, 19, or 20) ring atoms (i.e., 5- to 20-membered fused heterocyclyl). The fused heterocyclyl is preferably a fused heterocyclyl group having 6 to 14 ring atoms (i.e., 6- to 14-membered fused heterocyclyl), and more preferably a fused heterocyclyl group having 7 to 10 ring atoms (i.e., 7- to 10-membered fused heterocyclyl). The fused heterocyclyl includes bicyclic and polycyclic fused heterocyclyl (e.g., tricyclic fused heterocyclyl and tetracyclic fused heterocyclyl), preferably bicyclic fused heterocyclyl or tricyclic fused heterocyclyl, more preferably 3-membered/4-membered, 3-membered/5-membered, 3-membered/6-membered, 4-membered/4-membered, 4-membered/5-membered, 4-membered/6-membered, 5-membered/3-membered, 5-membered/4-membered, 5-membered/5-membered, 5-membered/6-membered, 5-membered/7-membered, 6-membered/3-membered, 6-membered/4-membered, 6-membered/5-membered, 6-membered/6-membered, 6-membered/7-membered, 7-membered/5-membered, or 7-membered/6-membered bicyclic fused heterocyclyl. Non-limiting examples include: 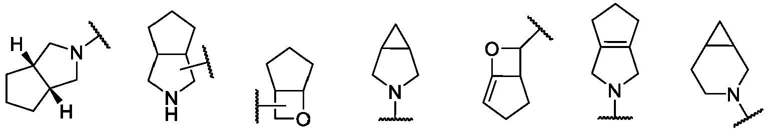 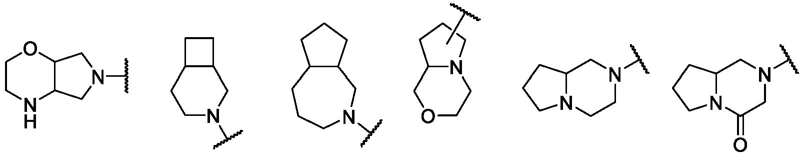 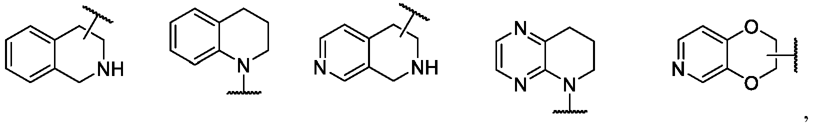 and the like.

The term "bridged heterocyclyl" refers to a polycyclic heterocyclic system in which two atoms that are not directly connected are shared between rings, which may contain in the rings one or more double bonds and contains in the rings at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-); and which has 5 to 20 (e.g., 5, 6, 7, 8, 9, 10, 11, 12, 13, 14, 15, 16, 17, 18, 19, or 20) ring atoms (i.e., 5- to 20-membered bridged heterocyclyl). The bridged heterocyclyl is preferably a bridged heterocyclyl group having 6 to 14 ring atoms (i.e., 6- to 14-membered bridged heterocyclyl), and more preferably a bridged heterocyclyl group having 7 to 10 ring atoms (i.e., 7- to 10-membered bridged heterocyclyl). According to the number of constituent rings, bridged heterocyclyl can be divided into bicyclic bridged heterocyclyl and polycyclic bridged heterocyclyl (e.g., tricyclic bridged heterocyclyl and tetracyclic bridged heterocyclyl), preferably bicyclic bridged heterocyclyl or tricyclic bridged heterocyclyl. Non-limiting examples include: 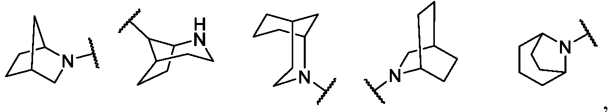 and the like.

Heterocyclyl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "aryl" refers to a monocyclic all-carbon aromatic ring (i.e., monocyclic aryl) or polycyclic aromatic ring system (i.e., polycyclic aryl) having a conjugated π-electron system, which has 6 to 14 (e.g., 6, 7, 8, 9, 10, 11, 12, 13, or 14) ring atoms (i.e., 6- to 14-membered aryl). The aryl is preferably an aryl group having 6 to 10 ring atoms (i.e., 6- to 10-membered aryl), and more preferably an aryl group having 8 to 10 ring atoms (i.e., 8-to 10-membered polycyclic aryl). An example of the monocyclic aryl is phenyl. Non-limiting examples of the polycyclic aryl include: naphthyl, anthryl, phenanthryl, and the like. The polycyclic aryl also includes those formed by fusing a phenyl group with one or more of a heterocyclyl group or a cycloalkyl group or fusing a naphthyl group with one or more of a heterocyclyl group or a cycloalkyl group, wherein the point of attachment is on the phenyl group or the naphthyl group, and in the circumstances the number of ring atoms continues to represent the number of ring atoms in the polycyclic aromatic ring system; non-limiting examples include: 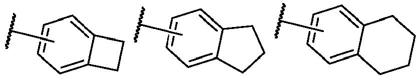 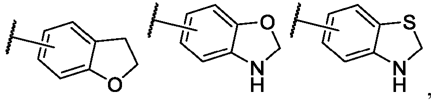 and the like.

Aryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, oxo, cyano, amino, nitro, cycloalkyl, heterocyclyl, aryl, and heteroaryl.

The term "heteroaryl" refers to a monocyclic heteroaromatic ring (i.e., monocyclic heteroaryl) or polycyclic heteroaromatic ring system (i.e., polycyclic heteroaryl) having a conjugated π-electron system, which contains in the ring(s) at least one (e.g., 1, 2, 3, or 4) heteroatom selected from the group consisting of nitrogen, oxygen, and sulfur (the nitrogen may be optionally oxidized to form a nitrogen oxide; the sulfur may be optionally substituted with oxo to form a sulfoxide or sulfone, excluding -O-O-, -O-S-, or -S-S-) and has 5 to 14 (e.g., 5, 6, 7, 8, 9, 10, 11, 12, 13, or 14) ring atoms (i.e., 5- to 14-membered heteroaryl). The heteroaryl is preferably a heteroaryl group having 5 to 10 ring atoms (i.e., 5- to 10-membered heteroaryl), more preferably a heteroaryl group having 5 or 6 ring atoms (i.e., 5- or 6-membered monocyclic heteroaryl), or preferably a heteroaryl group having 8 to 10 ring atoms (i.e., 8- to 10-membered polycyclic heteroaryl).

Non-limiting examples of the monocyclic heteroaryl include: furanyl, thienyl, thiazolyl, isothiazolyl, oxazolyl, isoxazolyl, oxadiazolyl, thiadiazolyl, imidazolyl, pyrazolyl, triazolyl, tetrazolyl, furazanyl, pyrrolyl, N-alkylpyrrolyl, pyridinyl, pyrimidinyl, pyridonyl, N-alkylpyridinone (e.g., 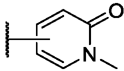 ), pyrazinyl, pyridazinyl, and the like.

Non-limiting examples of the polycyclic heteroaryl include: indolyl, indazolyl, quinolyl, isoquinolyl, quinoxalinyl, phthalazinyl, benzimidazolyl, benzothienyl, benzofuranyl, quinazolinyl, carbazolyl, pyrrolotriazinyl, 5,6,7,8-tetrahydro-triazolopyrazinyl, imidazopyridazinyl, [1,2,4]triazolo[1,5-a]pyridinyl, and the like. The polycyclic heteroaryl also includes those formed by fusing a monocyclic heteroaryl group with one or more aryl groups, wherein the point of attachment is on an aromatic ring, and in the circumstances the number of ring atoms continues to represent the number of ring atoms in the polycyclic heteroaromatic ring system. The polycyclic heteroaryl also includes those formed by fusing a monocyclic heteroaryl group with one or more of a cycloalkyl group or a heterocyclyl group, wherein the point of attachment is on the monocyclic heteroaromatic ring, and in the circumstances the number of ring atoms continues to represent the number of ring atoms in the polycyclic heteroaromatic ring system. Non-limiting examples include: 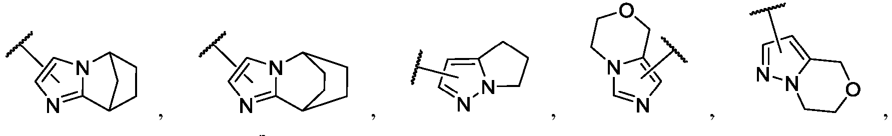 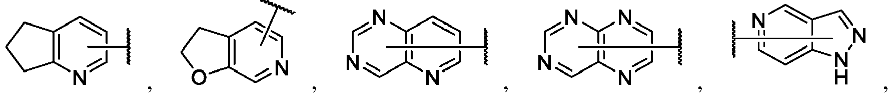 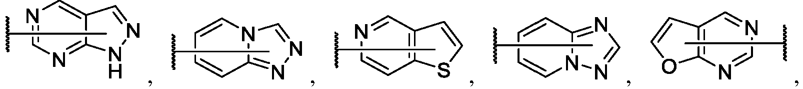 and the like.

Heteroaryl may be substituted or unsubstituted, and when it is substituted, it may be substituted at any accessible point of attachment, and the substituent is preferably selected from the group consisting of one or more of a D atom, halogen, alkyl, alkoxy, haloalkyl, haloalkoxy, cycloalkyloxy, heterocyclyloxy, hydroxy, hydroxyalkyl, cyano, amino, nitro, cycloalkyl, heterocyclyl, aryl, and heteroaryl.

The term "amino protecting group" refers to an easily removable group that is introduced onto an amino group in order for the amino group to remain unchanged when other parts of the molecule are involved in reactions. Non-limiting examples include: (trimethylsilyl)ethoxymethyl, tetrahydropyranyl, tert-butyloxycarbonyl (Boc), benzyloxycarbonyl (Cbz), fluorenylmethoxycarbonyl (Fmoc), allyloxycarbonyl (Alloc), trimethylsilylethoxycarbonyl (Teoc), methoxycarbonyl, ethoxycarbonyl, phthaloyl (Pht), p-toluenesulfonyl (Tos), trifluoroacetyl (Tfa), trityl (Trt), 2,4-dimethoxybenzyl (DMB), acetyl, benzyl, allyl, p-methoxybenzyl, and the like.

The term "hydroxy protecting group" refers to an easily removable group that is introduced onto a hydroxy group to block or protect the hydroxy group so that reactions take place on other functional groups of the compound. Non-limiting examples include: trimethylsilyl (TMS), triethylsilyl (TES), triisopropylsilyl (TIPS), tert-butyldimethylsilyl (TBS), tert-butyldiphenylsilyl (TBDPS), methyl, tert-butyl, allyl, benzyl, methoxymethyl (MOM), ethoxyethyl, 2-tetrahydropyranyl (THP), formyl, acetyl, benzoyl, p-nitrobenzoyl, and the like.

The term "cycloalkyloxy" refers to cycloalkyl-O-, wherein the cycloalkyl is as defined above.

The term "heterocyclyloxy" refers to heterocyclyl-O-, wherein the heterocyclyl is as defined above.

The term "aryloxy" refers to aryl-O-, wherein the aryl is as defined above.

The term "heteroaryloxy" refers to heteroaryl-O-, wherein the heteroaryl is as defined above.

The term "alkylthio" refers to alkyl-S-, wherein the alkyl is as defined above.

The term "haloalkyl" refers to alkyl substituted with one or more halogens, wherein the alkyl is as defined above.

The term "haloalkoxy" refers to an alkoxy group substituted with one or more halogens, wherein the alkoxy group is as defined above.

The term "deuterated alkyl" refers to alkyl substituted with one or more deuterium atoms, wherein the alkyl is as defined above.

The term "hydroxyalkyl" refers to alkyl substituted with one or more hydroxy groups, wherein the alkyl is as defined above.

The term "methylidene" refers to =CH₂.

The term "halogen" refers to fluorine, chlorine, bromine, or iodine.

The term "hydroxy" refers to -OH.

The term "sulfhydryl" refers to -SH.

The term "amino" refers to -NH₂.

The term "cyano" refers to -CN.

The term "nitro" refers to -NO₂.

The term "oxo" refers to "=O".

The term "carbonyl" refers to C=O.

The term "carboxyl" refers to -C(O)OH.

Ms refers to methanesulfonyl.

The term "carboxylate group" refers to -C(O)O(alkyl), -C(O)O(cycloalkyl), (alkyl)C(O)O-, or (cycloalkyl)C(O)O-, wherein the alkyl and cycloalkyl are as defined above.

The compounds of the present disclosure may exist in specific stereoisomeric forms. The term "stereoisomer" refers to isomers that are structurally identical but differ in the arrangement of the atoms in space. It includes cis and trans (or *Z* and *E*) isomers, (-)- and (+)-isomers, (*R*)- and (*S*)-enantiomers, diastereomers, (*D*)- and (*L*)-isomers, tautomers, atropisomers, conformers, and mixtures thereof (e.g., mixtures of racemates and diastereomers). Additional asymmetric atoms may be present in the substituents in the compounds of the present disclosure. All such stereoisomers and mixtures thereof are included within the scope of the present disclosure. Optically active (-)- and (+)-isomers, (*R*)- and (*S*)-enantiomers, and (*D*)- and (*L*)-isomers can be prepared by chiral synthesis, chiral reagents, or other conventional techniques. One isomer of a certain compound of the present disclosure may be prepared by asymmetric synthesis or with a chiral auxiliary, or, when the molecule contains a basic functional group (e.g., amino) or an acidic functional group (e.g., carboxyl), a diastereomeric salt is formed with an appropriate optically active acid or base, followed by diastereomeric resolution by conventional methods known in the art to give the pure isomer. In addition, separation of enantiomers and diastereomers is generally accomplished by chromatography.

In the chemical structures of the compounds of the present disclosure, a bond " 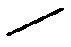 " represents an unspecified configuration; that is, if chiral isomers exist in the chemical structures, the bond " 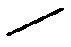 " may be " 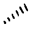 " or " 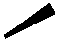 ", or both the configurations of " 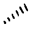 " and " 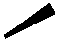 " are included simultaneously.

The compounds of the present disclosure may also exist in different tautomeric forms, and all such forms are included within the scope of the present disclosure. The term "tautomer" or "tautomeric form" refers to a structural isomer that exists in equilibrium and is readily converted from one isomeric form into another. It includes all possible tautomers; that is, it is present in the form of a single isomer or in the form of a mixture of the tautomers in any ratio. Non-limiting examples include: keto-enol, imine-enamine, lactam-lactim, and the like. An example of lactam-lactim in equilibrium is shown below:

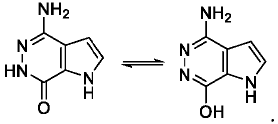

For example, reference to pyrazolyl is understood to include any one of the following two structures or a mixture of the two tautomers:

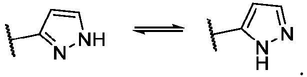

All tautomeric forms fall within the scope of the present disclosure, and the nomenclature of the compounds does not exclude any tautomer.

The compounds of the present disclosure include all suitable isotopic derivatives of the compounds thereof. The term "isotopic derivative" refers to a compound in which at least one atom is replaced with an atom having the same atomic number but a different atomic mass. Examples of isotopes that can be incorporated into the compounds of the present disclosure include stable and radioactive isotopes of hydrogen, carbon, nitrogen, oxygen, phosphorus, sulfur, fluorine, chlorine, bromine, iodine, etc., such as ²H (deuterium, D), ³H (deuterium, T), ¹¹C, ¹³C, ¹⁴C, ¹⁵N, ¹⁷O, ¹⁸O, ³²p, ³³p, ³³S, ³⁴S, ³⁵S, ³⁶S, ¹⁸F, ³⁶Cl, ⁸²Br, ¹²³I, ¹²⁴I, ¹²⁵I, ¹²⁹I, and ¹³¹I; deuterium is preferred.

Compared to non-deuterated drugs, deuterated drugs have the advantages of reduced toxic and side effects, increased drug stability, enhanced efficacy, prolonged biological halflives, and the like. All isotopic variations of the compounds of the present disclosure, whether radioactive or not, are intended to be included within the scope of the present disclosure. Each available hydrogen atom connected to a carbon atom may be independently replaced with a deuterium atom, wherein replacement of deuterium may be partial or complete, and replacement of partial deuterium refers to replacement of at least one hydrogen atom with at least one deuterium atom.

In the compounds of the present disclosure, when a position is specifically assigned "deuterium" or "D", the position should be construed as the abundance of deuterium being at least 1000 times greater than the natural abundance of deuterium (which is 0.015%) (i.e., at least 15% deuterium incorporation). In some embodiments, the abundance of deuterium of each of the assigned deuterium atoms is at least 1000 times greater than the natural abundance of deuterium (i.e., at least 15% deuterium incorporation). In some embodiments, the abundance of deuterium of each of the assigned deuterium atoms is at least 2000 times greater than the natural abundance of deuterium (i.e., at least 30% deuterium incorporation). In some embodiments, the abundance of deuterium of each of the assigned deuterium atoms is at least 3000 times greater than the natural abundance of deuterium (i.e., at least 45% deuterium incorporation). In some embodiments, the abundance of deuterium of each of the assigned deuterium atoms is at least 3340 times greater than the natural abundance of deuterium (i.e., at least 50.1% deuterium incorporation). In some embodiments, the abundance of deuterium of each of the assigned deuterium atoms is at least 3500 times greater than the natural abundance of deuterium (i.e., at least 52.5% deuterium incorporation). In some embodiments, the abundance of deuterium of each of the assigned deuterium atoms is at least 4000 times greater than the natural abundance of deuterium (i.e., at least 60% deuterium incorporation). In some embodiments, the abundance of deuterium of each of the assigned deuterium atoms is at least 4500 times greater than the natural abundance of deuterium (i.e., at least 67.5% deuterium incorporation). In some embodiments, the abundance of deuterium of each of the assigned deuterium atoms is at least 5000 times greater than the natural abundance of deuterium (i.e., at least 75% deuterium incorporation). In some embodiments, the abundance of deuterium of each of the assigned deuterium atoms is at least 5500 times greater than the natural abundance of deuterium (i.e., at least 82.5% deuterium incorporation). In some embodiments, the abundance of deuterium of each of the assigned deuterium atoms is at least 6000 times greater than the natural abundance of deuterium (i.e., at least 90% deuterium incorporation). In some embodiments, the abundance of deuterium of each of the assigned deuterium atoms is at least 6333.3 times greater than the natural abundance of deuterium (i.e., at least 95% deuterium incorporation). In some embodiments, the abundance of deuterium of each of the assigned deuterium atoms is at least 6466.7 times greater than the natural abundance of deuterium (i.e., at least 97% deuterium incorporation). In some embodiments, the abundance of deuterium of each of the assigned deuterium atoms is at least 6600 times greater than the natural abundance of deuterium (i.e., at least 99% deuterium incorporation). In some embodiments, the abundance of deuterium of each of the assigned deuterium atoms is at least 6633.3 times greater than the natural abundance of deuterium (i.e., at least 99.5% deuterium incorporation).

"Optional" or "optionally" means that the event or circumstance subsequently described may, but does not necessarily, occur and includes an instance where the event or circumstance occurs and an instance where it does not. For example, "alkyl that is optionally substituted with halogen or cyano" includes an instance where the alkyl is substituted with halogen or cyano and an instance where the alkyl is not substituted with halogen or cyano.

"Substitution" or "substituted" means that one or more, preferably 1-6, and more preferably 1-3, hydrogen atoms in the group are independently substituted with a corresponding number of substituents. Those skilled in the art can determine (experimentally or theoretically) possible or impossible substitutions without undue effort. For example, it may be unstable when an amino or hydroxy group having free hydrogen binds to a carbon atom having an unsaturated bond (e.g., an olefin).

"Pharmaceutical composition" refers to a mixture containing one or more of the compounds or the pharmaceutically acceptable salts thereof described herein, and other chemical components, and other components, for example, pharmaceutically acceptable carriers and excipients. The pharmaceutical composition is intended to promote administration to an organism and facilitate the absorption of the active ingredient so that it can exert its biological activity.

"Pharmaceutically acceptable salt" refers to a salt of the compound of the present disclosure, which may be selected from the group consisting of inorganic or organic salts. Such salts are safe and effective when used in the body of a mammal and possess the requisite biological activity. The salts may be prepared separately during the final separation and purification of the compound, or by reacting an appropriate group with an appropriate base or acid. Bases commonly used to form pharmaceutically acceptable salts include inorganic bases such as sodium hydroxide and potassium hydroxide, and organic bases such as ammonia. Acids commonly used to form pharmaceutically acceptable salts include inorganic acids and organic acids.

For drugs or pharmacologically active agents, the term "therapeutically effective amount" refers to an amount of the drug or agent sufficient to achieve, or at least partially achieve, the desired effect. The determination of the therapeutically effective amount varies from person to person. It depends on the age and general condition of a subject, as well as the specific active substance used. The appropriate therapeutically effective amount in a case may be determined by those skilled in the art in the light of routine tests.

The term "pharmaceutically acceptable" as used herein means that those compounds, materials, compositions, and/or dosage forms that are, within the scope of reasonable medical judgment, suitable for use in contact with the tissues of patients without excessive toxicity, irritation, allergic reaction, or other problems or complications, and are commensurate with a reasonable benefit/risk ratio and effective for the intended use. As used herein, the singular forms "a", "an" and "the" include plural references and vice versa, unless otherwise clearly defined in the context.

When the term "about" is applied to parameters such as pH, concentration, and temperature, it means that the parameter may vary by ±10%, and sometimes more preferably within ±5%. As will be understood by those skilled in the art, when the parameters are not critical, the numbers are generally given for illustrative purposes only and are not intended to be limiting.

### Synthesis Methods for Compounds of the Present Disclosure

To achieve the purposes of the present disclosure, the following technical solutions are adopted in the present disclosure:

### Scheme 1

A method for preparing the compound of general formula (I) or the pharmaceutically acceptable salt thereof of the present disclosure, comprising the following step: 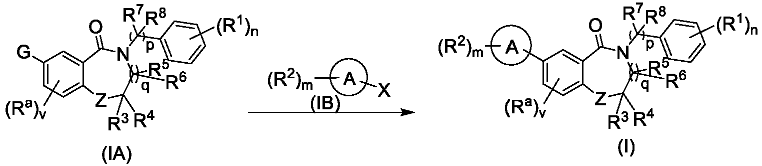 conducting a suzuki coupling reaction of a compound of general formula (IA) or a salt thereof with a compound of general formula (IB) or a salt thereof under alkaline conditions in the presence of a catalyst to give the compound of general formula (I) or the pharmaceutically acceptable salt thereof;
wherein:
G is 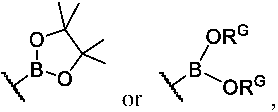 and R^{G} is a hydrogen atom or C₁₋₆ alkyl;
X is halogen, preferably Br;
ring A, R¹ to R⁸, Z, R^{a}, v, p, q, m and n are as defined in general formula (I).

### Scheme 2

A method for preparing the compound of general formula (II) or the pharmaceutically acceptable salt thereof of the present disclosure, comprising the following step: 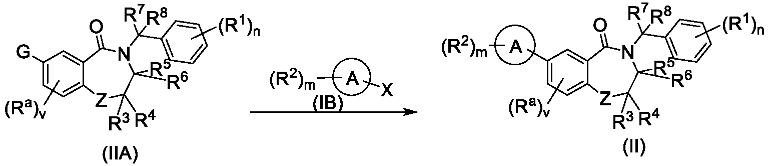 conducting a suzuki coupling reaction of a compound of general formula (IIA) or a salt thereof with a compound of general formula (IB) or a salt thereof under alkaline conditions in the presence of a catalyst to give the compound of general formula (II) or the pharmaceutically acceptable salt thereof; wherein:
G is 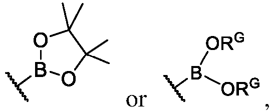 and R^{G} is a hydrogen atom or C₁₋₆ alkyl;
X is halogen, preferably Br;
ring A, R¹ to R⁸, Z, R^{a}, v, m and n are as defined in general formula (II).

### Scheme 3

A method for preparing the compound of general formula (III) or the salt thereof of the present disclosure, comprises the following step: 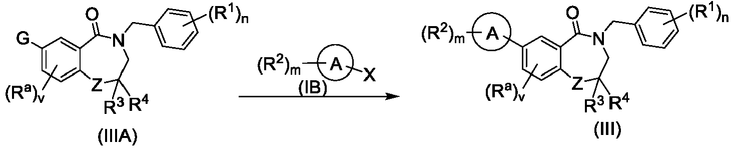 conducting a suzuki coupling reaction of a compound of general formula (IIIA) or a salt thereof with a compound of general formula (IB) or a salt thereof under alkaline conditions in the presence of a catalyst to give the compound of general formula (III) or the pharmaceutically acceptable salt thereof;
wherein:
G is 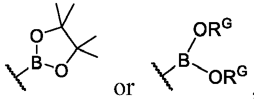 , and R^{G} is a hydrogen atom or C₁₋₆ alkyl;
X is halogen, preferably Br;
ring A, R¹ to R⁴, Z, R^{a}, v, m and n are as defined in general formula (III).

### Scheme 4

A method for preparing the compound of general formula (IV) or the pharmaceutically acceptable salt thereof of the present disclosure, comprising the following step: 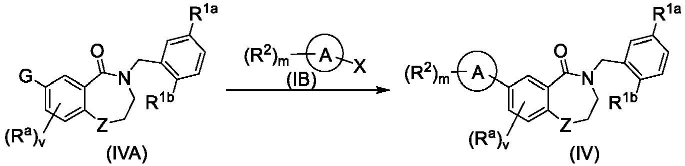 conducting a suzuki coupling reaction of a compound of general formula (IVA) or a salt thereof with a compound of general formula (IB) or a salt thereof under alkaline conditions in the presence of a catalyst to give the compound of general formula (IV) or the pharmaceutically acceptable salt thereof;
wherein:
G is 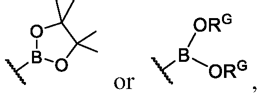 and R^{G} is a hydrogen atom or C₁₋₆ alkyl;
X is halogen, preferably Br;
ring A, R^{1a}, R^{1b}, R², R^{a}, v, m and Z are as defined in general formula (IV).

### Scheme 5

A method for preparing the compound of general formula (V) or the pharmaceutically acceptable salt thereof of the present disclosure, comprising the following step: 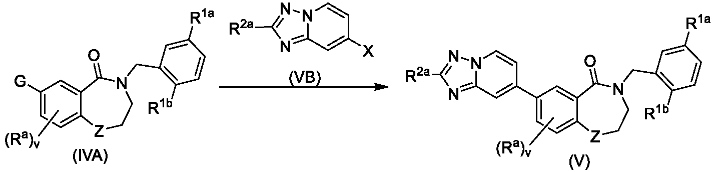 conducting a suzuki coupling reaction of a compound of general formula (IVA) or a salt thereof with a compound of general formula (VB) or a salt thereof under alkaline conditions in the presence of a catalyst to give the compound of general formula (V) or the pharmaceutically acceptable salt thereof;
wherein:
G is 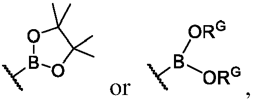 and R^{G} is a hydrogen atom or C₁₋₆ alkyl;
X is halogen, preferably Br;
R^{1a}, R^{1b}, R^{a}, v, R^{2a} and Z are as defined in general formula (V).

The suzuki coupling reaction described in the above synthesis schemes is preferably carried out under alkaline conditions (e.g., potassium carbonate) and in the presence of a metal catalyst, wherein the metal catalyst is preferably selected from the group consisting of [1,1'-bis(di-*tert*-butylphosphino)ferrocene]palladium(II) dichloride, [1,1'-bis(diphenylphosphino)ferrocene]palladium(II) dichloride and [1,1'-bis(diphenylphosphino)ferrocene]palladium(II) dichloride dichloromethane complex, more preferably [1,1'-bis(di-*tert*-butylphosphino)ferrocene]palladium(II) dichloride. The reagents providing the alkaline conditions in the above synthesis schemes include organic bases and inorganic bases, wherein the organic bases include, but are not limited to, triethylamine, pyridine, *N,N*-diisopropylethylamine, *n*-butyllithium, lithium diisopropylamide, sodium acetate, potassium acetate, sodium *tert*-butoxide, potassium tert-butoxide, or 1,8-diazabicycloundec-7-ene, and the inorganic bases include, but are not limited to, sodium hydride, potassium phosphate, sodium carbonate, potassium carbonate, cesium carbonate, cadmium carbonate, sodium hydroxide, lithium hydroxide monohydrate, lithium hydroxide, and potassium hydroxide; preferably, the reagent for the alkaline condition is potassium carbonate.

The reaction of the above steps is preferably conducted in a solvent including, but not limited to: pyridine, ethylene glycol dimethyl ether, acetic acid, methanol, ethanol, acetonitrile, *n*-butanol, toluene, tetrahydrofuran, dichloromethane, petroleum ether, ethyl acetate, *n*-hexane, dimethyl sulfoxide, 1,4-dioxane, water, *N,N-*dimethylformamide, *N,N-*dimethylacetamide, 1,2-dibromoethane, and a mixture thereof.

### DETAILED DESCRIPTION

The present disclosure is further described below with reference to examples, but these examples are not intended to limit the scope of the present disclosure.

### Examples

The structures of the compounds were determined by nuclear magnetic resonance (NMR) spectroscopy or/and mass spectrometry (MS). NMR shifts (δ) are given in a unit of 10⁻⁶ (ppm). NMR analysis was performed on a Bruker AVANCE-400 nuclear magnetic resonance instrument, with dimethyl sulfoxide-D6 (DMSO-*d₆*), chloroform-D (CDCl₃) and methanol-D4 (CD₃OD) as solvents and tetramethylsilane (TMS) as an internal standard.

The MS analyses were performed on an Agilent 1200/1290 DAD-6110/6120 Quadrupole MS liquid chromatography-mass spectrometry system (manufacturer: Agilent; MS model: 6110/6120 Quadrupole MS), Waters ACQuity UPLC-QD/SQD (manufacturer: Waters; MS model: Waters ACQuity Qda Detector/Waters SQ Detector) and THERMO Ultimate 3000-Q Exactive (manufacturer: THERMO; MS model: THERMO Q Exactive).

The high performance liquid chromatography (HPLC) analyses were performed on Agilent HPLC 1200DAD, Agilent HPLC 1200VWD, and Waters HPLC e2695-2489 high performance liquid chromatographs.

The chiral HPLC analyses were performed on an Agilent 1260 DAD high performance liquid chromatograph.

The preparative high performance liquid chromatography steps were performed on Waters 2545-2767, Waters 2767-SQ Detecor2, Shimadzu LC-20AP, and Gilson GX-281 preparative chromatographs.

The preparative chiral chromatography steps were performed on a Shimadzu LC-20AP preparative chromatograph.

The CombiFlash preparative flash chromatograph used was Combiflash Rf200 (TELEDYNE ISCO).

The thin-layer chromatography silica gel plates used were Yantai Huanghai HSGF254 or Qingdao GF254 silica gel plates. The silica gel plates used in the thin-layer chromatography (TLC) steps had a layer thickness of 0.15 mm-0.2 mm, and those used in the thin-layer chromatography separation and purification had a layer thickness of 0.4 mm-0.5 mm.

In the silica gel column chromatography steps, a 200-300 mesh silica gel (Huanghai, Yantai) was generally used as the carrier.

The kinase mean inhibition rates and IC₅₀ values were measured using a NovoStar microplate reader (BMG, Germany).

The known starting materials in the present disclosure may be synthesized by using or following methods known in the art, or may be purchased from companies such as ABCR GmbH & Co. KG, Acros Organics, Aldrich Chemical Company, Accela ChemBio Inc., and Chembee Chemicals.

In the examples, the reactions can all be performed under an argon atmosphere or a nitrogen atmosphere unless otherwise specified.

The argon atmosphere or nitrogen atmosphere means that the reaction flask is connected to a balloon containing about 1 L of argon or nitrogen gas.

The hydrogen atmosphere means that the reaction flask is connected to a balloon containing about 1 L of hydrogen gas.

The pressurized hydrogenation reactions were performed using a Parr 3916EKX hydrogenator and a Qinglan QL-500 hydrogenator, or an HC2-SS hydrogenator.

The hydrogenation reactions generally involved 3 rounds of vacuumization and hydrogen filling.

The microwave reactions were performed using a CEM Discover-S 908860 microwave reactor.

In the examples, the solutions were aqueous solutions unless otherwise specified.

In the examples, the reaction temperature was room temperature, i.e., 20 °C-30 °C, unless otherwise specified.

The reaction process monitoring in the examples was performed using thin-layer chromatography (TLC). The developing solvents used in the reactions, the eluent systems used in the column chromatography purification and the developing solvent systems used in the thin-layer chromatography include: A: n-hexane/ethyl acetate system, and B: dichloromethane/methanol system. The volume ratios of the solvents were adjusted depending on the polarity of the compounds, or by adding a small amount of basic or acidic reagents such as triethylamine and acetic acid.

### Example 1

### 7-(2-Amino-[1,2,4]triazolo[1,5-a]pyridin-7-yl)-4-(2-fluoro-5-(trifluoromethoxy)benzyl)-3,4-dihydrobenzo[f][1,4]oxazepin-5(2H)-one 1

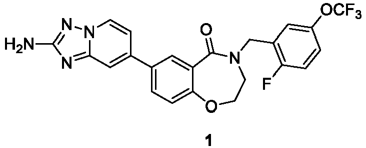

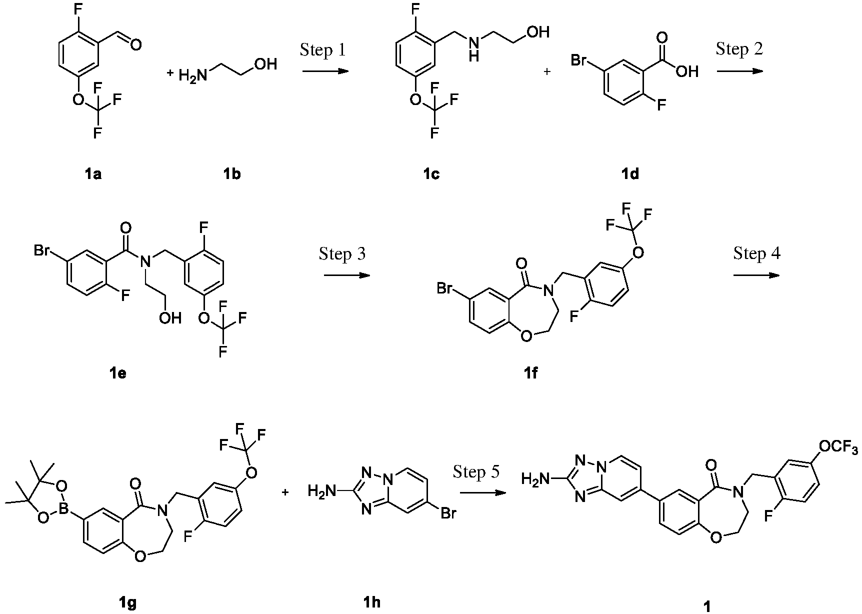

### Step 1

### 2-((2-Fluoro-5-(trifluoromethoxy)benzyl)amino)ethan-1-ol 1c

2-Fluoro-5-(trifluoromethoxy)benzaldehyde **1a** (1.50 g, 7.21 mmol, prepared by the method in Example 49 disclosed in the patent application US2006128691A1) and ethanolamine **1b** (660 mg, 10.80 mmol) were dissolved in a methanol solution (30 mL), and then a drop of glacial acetic acid was added dropwise. The mixture was stirred at room temperature for 5 h, and sodium borohydride (546 mg, 14.43 mmol) was added. The resulting mixture was stirred at room temperature for 1 h and concentrated under reduced pressure, and the residue was purified by silica gel column chromatography with eluent system A to give the title product **1c** (1.2 g, yield: 65.76%).

MS m/z (ESI): 254.0 [M+1].

### Step 2

### 5-Bromo-2-fluoro-N-(2-fluoro-5-(trifluoromethoxy)benzyl)-N-(2-hydroxyethyl)benzamide 1e

**1c** (220 mg, 0.87 mmol) and 5-bromo-2-fluorobenzoic acid **1d** (200 mg, 0.91 mmol, Adamas) were dissolved in *N,N*-dimethylformamide (10 mL), and then *N,N-*diisopropylethylamine (350 mg, 2.70 mmol) and 2-(7-azobenzotriazole)-*N,N,N',N'*'-tetramethyluronium hexafluorophosphate (500 mg, 1.32 mmol) were added. The reaction solution was stirred at room temperature for 2 h and poured into water (20 mL), and the resulting mixture was extracted with ethyl acetate (20 mL × 3). The organic phases were combined, washed with water (20 mL) and saturated sodium chloride solution (20 mL) in sequence, dried over anhydrous sodium sulfate, and filtered to remove the drying agent. The filtrate was concentrated under reduced pressure, and the residue was purified by silica gel column chromatography with eluent system A to give the title product **1e** (210 mg, yield: 50.6%).

MS m/z (ESI): 454.1 [M+1].

### Step 3

### 7-Bromo-4-(2-fluoro-5-(trifluoromethoxy)benzyl)-3,4-dihydrobenzo[f][1,4]oxazepin-5(2H)-one 1f

**1e** (210 mg, 0.46 mmol) was dissolved in *N,N*-dimethylformamide (10 mL), and the mixture was cooled to 0-5 °C in an ice bath. Sodium hydride (50 mg, 1.25 mmol, 60%) was added portionwise, and the mixture was warmed to room temperature and reacted for 1.5 h. The reaction solution was slowly poured into ice water (20 mL) to quench the reaction, and the resulting mixture was extracted with ethyl acetate (20 mL × 2). The organic phases were combined, washed with water (20 mL) and saturated sodium chloride solution (20 mL) in sequence, dried over anhydrous sodium sulfate, and filtered to remove the drying agent. The filtrate was concentrated under reduced pressure, and the residue was purified by silica gel column chromatography with eluent system A to give the title product **1f** (110 mg, yield: 54.8%).

MS m/z (ESI): 434.1 [M+1].

### Step 4

### 4-(2-Fluoro-5-(trifluoromethoxy)benzyl)-7-(4,4,5,5-tetramethyl-1,3,2-dioxaborolan-2-yl)-3,4-dihydrobenzo[f][1,4]oxazepin-5(2H)-one 1g

**1f** (110 mg, 0.25 mmol), bis(pinacolato)diboron (85 mg, 0.33 mmol), potassium acetate (85 mg, 0.87 mmol) and [1,1'-bis(diphenylphosphino)ferrocene]palladium(II) dichloride (15 mg, 0.021 mmol) were dissolved in 1,4-dioxane (10 mL), and the mixture was reacted at 90 °C for 2 h under a nitrogen atmosphere and concentrated under reduced pressure. Water (10 mL) was added, and the resulting mixture was extracted with dichloromethane (10 mL × 3). The organic phases were combined, washed with water (10 mL) and saturated sodium chloride solution (10 mL) in sequence, dried over anhydrous sodium sulfate, and filtered to remove the drying agent. The filtrate was concentrated under reduced pressure to give the title product **1g** (120 mg), which was directly used in the next step without purification.

MS m/z (ESI): 482.3 [M+1].

### Step 5

### 7-(2-Amino-[1,2,4]triazolo[1,5-a]pyridin-7-yl)-4-(2-fluoro-5-(trifluoromethoxy)benzyl)-3,4-dihydrobenzo[f][1,4]oxazepin-5(2H)-one 1

**1g** (110 mg, 0.23 mmol) and 7-bromo-[1,2,4]triazolo[1,5-*a*]pyridin-2-amine **1h** (60 mg, 0.28 mmol) were dissolved in 1,4-dioxane (10 mL) and water (4 mL), and then [1,1'-bis(di-*tert*-butylphosphino)ferrocene]palladium(II) dichloride (15 mg, 0.019 mmol, admas) and potassium carbonate (100 mg, 0.72 mmol) were added. Under a nitrogen atmosphere, the reaction solution was heated to 90 °C and stirred for 2 h. The reaction solution was cooled to room temperature and concentrated under reduced pressure to remove most of the solvent, water (20 mL) was added to the reaction solution, and the resulting mixture was extracted with ethyl acetate (20 mL × 3). The organic phase was washed with water (20 mL) and saturated sodium chloride solution (20 mL) in sequence, dried over anhydrous sodium sulfate, and filtered to remove the drying agent. The filtrate was concentrated under reduced pressure, and the residue was purified by silica gel column chromatography with eluent system B to give the title product **1** (35 mg, yield: 31.4%).

MS m/z (ESI): 488.4 [M+1].

¹H NMR (500MHz, DMSO-*d₆*) *δ* 8.58-8.56 (m,1H), 8.06 (s,1H), 7.96-7.94 (m,1H), 7.61(s,1H), 7.41-7.38 (m,3H), 7.19-7.16 (m, 2H), 6.04 (brs, 2H), 4.86 (s, 2H), 4.35-4.33(m, 2H), 3.71-3.69 (m, 2H).

### Example 2

### 8-(2-Amino-[1,2,4]triazolo[1,5-a]pyridin-7-yl)-2-(2-fluoro-5-(trifluoromethoxy)benzyl)-2,3,4,5-tetrahydro-1H-benzo[c]azepin-1-one 2

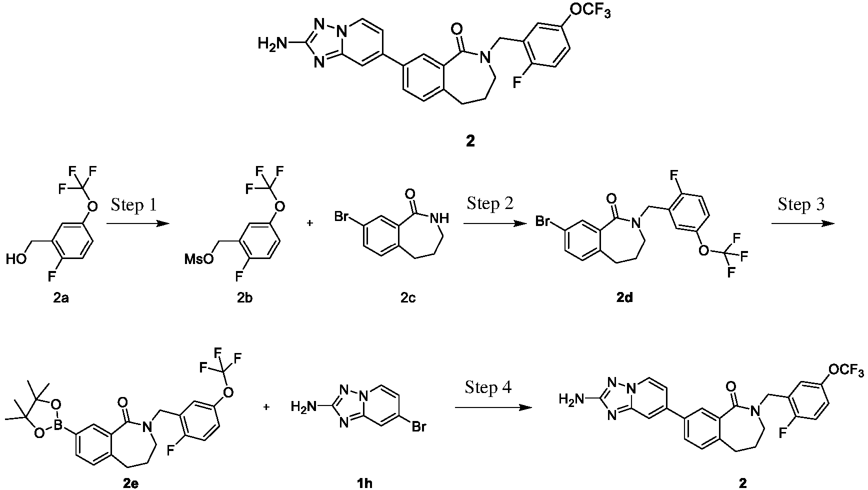

### Step 1

### 2-Fluoro-5-(trifluoromethoxy)benzyl methanesulfonate 2b

(2-Fluoro-5-(trifluoromethoxy)phenyl)methanol **2a** (750 mg, 3.57 mmol) and triethylamine (1.09 g, 10.77 mmol) were dissolved in dichloromethane (20 mL), and then methanesulfonyl chloride (614 mg, 5.36 mmol) was added slowly and dropwise at room temperature. The reaction solution was stirred at room temperature for 0.5 h. The reaction solution was diluted with dichloromethane (20 mL), washed with water (20 mL) and saturated sodium chloride solution (20 mL) in sequence, dried over anhydrous sodium sulfate, and filtered to remove the drying agent. The filtrate was concentrated under reduced pressure to give the title product **2b** (1.0 g, yield: 97.2%).

### Step 2

### 8-Bromo-2-(2-fluoro-5-(trifluoromethoxy)benzyl)-2,3,4,5-tetrahydro-1H-benzo[c]azepin-1-one 2d

8-Bromo-2,3,4,5-tetrahydro-1H-2-benzazepin-1-one **2c** (175 mg, 0.73 mmol) was dissolved in tetrahydrofuran (10 mL), and then sodium hydride (56 mg, 1.46 mmol, 60%) was added at room temperature. The reaction solution was stirred at room temperature for 1 h, followed by the dropwise addition of a solution of compound **2b** (315 mg, 1.09 mmol) in tetrahydrofuran (1 mL). The reaction solution was stirred at room temperature for 1 h and poured into water (30 mL), and the resulting mixture was extracted with ethyl acetate (40 mL × 3). The organic phases were combined, washed with water (20 mL) and saturated sodium chloride solution (20 mL) in sequence, dried over anhydrous sodium sulfate, and filtered to remove the drying agent. The filtrate was concentrated under reduced pressure, and the residue was purified by silica gel column chromatography with eluent system A to give the title product **2d** (300 mg, yield: 95.2 %).

MS m/z (ESI): 432.0 [M+1].

### Step 3

### 2-(2-Fluoro-5-(trifluoromethoxy)benzyl)-8-(4,4,5,5-tetramethyl-1,3,2-dioxaborolan-2-yl)-2,3,4,5-tetrahydro-1H-benzo[c]azepan-1-one 2e

**2d** (300 mg, 0.69 mmol), bis(pinacolato)diboron (250 mg, 0.98 mmol), potassium acetate (240 mg, 2.44 mmol) and [1,1'-bis(diphenylphosphino)ferrocene]palladium(II) dichloride (51 mg, 0.07 mmol) were dissolved in 1,4-dioxane (20 mL), and the mixture was reacted at 90 °C for 2 h under a nitrogen atmosphere and concentrated under reduced pressure. Water (20 mL) was added, and the resulting mixture was extracted with dichloromethane (20 mL × 3). The organic phases were combined, washed with water (10 mL) and saturated sodium chloride solution (10 mL) in sequence, dried over anhydrous sodium sulfate, and filtered to remove the drying agent. The filtrate was concentrated under reduced pressure to give the title product **2e** (332 mg, yield: 99.8%), which was directly used in the next step without purification.

MS m/z (ESI): 480.3 [M+1].

### Step 4

### 8-(2-Amino-[1,2,4]triazolo[1,5-a]pyridin-7-yl)-2-(2-fluoro-5-(trifluoromethoxy)benzyl)-2,3,4,5-tetrahydro-1H-benzo[c]azepin-1-one 2

**2e** (332 mg, 0.69 mmol) and compound **1h** (178 mg, 0.84 mmol) were dissolved in 1,4-dioxane (20 mL) and water (5 mL), and then [1,1'-bis(di-*tert-*butylphosphino)ferrocene]palladium(II) dichloride (52 mg, 0.07 mmol, adamas) and potassium carbonate (288 mg, 2.08 mmol) were added. Under a nitrogen atmosphere, the reaction solution was heated to 90 °C and stirred for 3 h. The reaction solution was cooled to room temperature and concentrated under reduced pressure to remove most of the solvent, water (20 mL) was added to the reaction solution, and the resulting mixture was extracted with ethyl acetate (20 mL × 3). The organic phase was washed with water (20 mL) and saturated sodium chloride solution (20 mL) in sequence, dried over anhydrous sodium sulfate, and filtered to remove the drying agent. The filtrate was concentrated under reduced pressure, and the residue was purified by silica gel column chromatography with eluent system B to give the title product **2** (150 mg, yield: 44.6%).

MS m/z (ESI): 486.5 [M+1].

¹H NMR (500MHz, DMSO-*d₆*) *δ* 8.59-8.58 (d,1H), 7.90-7.87(m,2H), 7.65 (s,1H), 7.48-7.37 (m,4H), 7.22-7.21 (d,1H), 6.05 (s, 2H), 4.81 (s, 2H), 3.28-3.26 (m, 2H), 3.73-3.70 (m, 2H), 1.83-1.77 (m, 2H).

### Biological Evaluations

### Test Example 1: Inhibitory Effects of Compounds of the Present Disclosure on Necroptosis of HT29 Cells

The CTG method was used for detecting the inhibitory effects of the compounds of the present disclosure on the necroptosis of HT29 cells. The specific procedures are as follows:

### 1. Reagents and instruments

### 1.1 Reagents

| Material name | Cat. No. | Manufacturer |
|---|---|---|
| McCoy's 5A Medium | 16600-082 | Gibco |
| DPBS | 14190-144 | Gibco |
| 0.25% Trypsin-EDTA (1×) | 25200-072 | Gibco |
| Fetal Bovine Serum (FBS) | 10091-148 | Gibco |
| Z-VAD-FMK | A12373 | AdooQ BioScience |
| AT-406 | A11163 | AdooQ BioScience |
| Recombinant Human TNF-α Protein | 210-TA-020 | R&D systems |
| Countstar Cell Counting Plate | 12-0005-50 | Countstar |
| 75 cm² Cell Culture Flask with Vent Cap | 430641 | Coring |
| Corning^{®} 96 Well Black Polystyrene Microplate | CLS3603 | Coring |
| CellTiter-Glo^{®} Luminescent Cell Viability Assay | G7572 | Promega |

### 1.2 Instruments

| Instrument name | Manufacturer | Model information |
|---|---|---|
| Automated cell counter | Countstar | IC1000 |
| Hood | Thermo | 1300AII |
| Thermo Incubator | Thermo | I160 |
| PHERASTAR HS Microplate reader | BMG LABTECH | PHERASTAR HS |
| Centrifuge | Beckman coulter | Allegra X-12 centrifuge |

### 2. Cell and culture method

HT29 cells were purchased from American Type Culture Collection (ATCC, HTB-38) and cultured with a McCoy's 5A medium (10% FBS) in a 37 °C incubator with 5% carbon dioxide. The cell culture density was maintained at 2 × 10⁴ to 8 × 10⁴ cells/cm², and the cells were passaged 2-3 times a week.

### 3. Compound preparation

a. Test compounds were dissolved in DMSO to 2 mM.
b. The compounds were 3-fold diluted from an initial concentration of 2 mM to 10 gradient concentrations.
c. All concentrations of compounds were 100-fold diluted with the culture medium for later use.

### 4. Experimental procedure

a. The cells were counted, and the cell density was adjusted to 1 × 10⁵ cells/mL with a fresh culture medium.
b. The cells were seeded in a 96-well plate at 80 µL/well and cultured at 37 °C overnight.
c. 10 µL of the compound (10 µL of 0.5% DMSO was added in columns 1 and 12) was added to each well, and the cells were cultured at 37 °C for 1 h.
d. TZA mixture (TNFα 0.2 µg/mL, Z-VAD-FMK 2 mM, AT-406 0.1 mM) was prepared using the culture medium, 10 µL of the TZA mixture (10 µL of the culture medium was added to column 1 as a control) was added to each well, and the cells were cultured at 37 °C for 24 h.
e. CellTiter-Glo reagent was left to stand at room temperature for 30 min, 50 µL of the CellTiter-Glo reagent was added to each well, and the plate was shaken at 850 rpm at room temperature for 2 min.
f. After the culture plate was left to stand at room temperature for 0.5 h, a stable luminescence signal was obtained, and then the plate was read using a microplate reader. g. The IC₅₀ values of the compounds of the present disclosure were calculated by the Graphpad software.

**Table 1. Inhibitory effects of compounds of the present disclosure on necroptosis of HT29 cells**

| Example No. | IC₅₀ (nM) |
|---|---|
| 1 | 13.3 |
| 2 | 1.5 |

Conclusion: the compounds of the present disclosure have significant inhibitory effects on the necroptosis of HT29 cells.

### Test Example 2: Inhibitory Activities of Compounds of the Present Disclosure Against RIPK1 Kinase (ADP-Glo Method)

The ADP GLO method was used for detecting the inhibitory activities of the compounds of the present disclosure against RIPK1 kinase. The method is specifically as follows:

### 1. Reagents and instruments

1) RIPK1 kinase reaction system (containing RIPK1 kinase, MBP, 5× reaction buffer, MnCl₂ and DTT) (Promega, VA7592)
2) ADP-Glo kinase detection kit (containing ADP-Glo reagent and kinase detection reagents) (Promega, V9101)
3) ATP solution (10 mM) (Thermofisher PV3227)
4) 96-well small-volume white plate (Cisbio, 66PL96100)
5) PHERA star microplate reader (BMG labtech)

### 2. Experimental method

### 2.1 Reagent preparation

a. Reaction buffer: a reaction buffer containing DTT at a final concentration of 50 µM and MnCl₂ at a final concentration of 4 mM was prepared using 5× reaction buffer;
b. RIPK1 kinase solution: a RIPK1 kinase solution at a final concentration of 10 ng/µL was prepared using the reaction buffer;
c. Mixed substrate of ATP and MBP: ATP at a final concentration of 60 µM and MBP at a final concentration of 0.6 µg/µL were separately prepared using the reaction buffer, and the prepared ATP and MBP were mixed in equal volumes;
d. Compound: the compound was 3-fold diluted from an initial concentration of 33.3 µM to 9 gradient concentrations. All concentrations of compounds were 33.3-fold diluted with the reaction buffer for later use.

### 2.2 Experimental procedure

a. The prepared RIPK1 enzyme solution was added to a 96-well plate at 2 µL/well, and 2 µL of the reaction buffer was added to column 1 instead of the enzyme.
b. 2 µL of the compound was added to each well, DMSO was used as a control and added to column 1 and the last column instead of the compound, and the plate was centrifuged, shaken for 2 min, and incubated at room temperature for 10 min.
c. 2 µL of the mixed substrate of ATP and MBP was added to each well, and the plate was centrifuged, shaken for 2 min, and incubated at room temperature for 90 min.
d. 6 µL of the ADP-Glo reagent was added to each well, and the plate was centrifuged, shaken for 2 min, and incubated at room temperature for 40 min.
e. 12 µL of the kinase detection reagent was added to each well, and the plate was centrifuged, shaken for 2 min, and incubated at room temperature for 40 min.
f. The plate was read using the PHERA star microplate reader, and relative luminescence unit (RLU) values were recorded.
g. Plotting was performed using the Graphpad software, and the IC₅₀ values of the compounds of the present disclosure were calculated.

**Table 2: Inhibitory activities of compounds of the present disclosure against RIPK1 kinase**

| Example No. | IC₅₀ (nM) |
|---|---|
| 1 | 23 |
| 2 | 2.7 |

Conclusion: the compounds of the present disclosure have significant inhibitory activities against the RIPK1 kinase.

### Test Example 3: Pharmacokinetic Evaluation of Compound of the Present Disclosure in Dogs

### 1. Abstract

Beagles were used as test animals. Plasma concentrations of the compound of Example 2 at different time points after intragastric administration of Example 2 compound to beagles were determined using an LC/MS/MS method. The pharmacokinetic performance of the compound of the present disclosure was studied in beagles and its pharmacokinetic profile was evaluated.

### 2. Experimental protocol

### 2.1. Experimental compound

The compound of Example 2.

### 2.2. Test animals

Eight beagles were provided by Jiangsu Johnbio Biotechnology Co., Ltd., with an equal number of males and females. The production license number was SCXK (Jiangsu) 2018-0005.

### 2.3. Compound solution preparation

A certain amount of the compound of Example 2 was weighed out, and 5% DMSO + 30% PG + 30% PEG400 + 35% normal saline were added to prepare a 0.4 mg/mL clear solution.

### 2.4. Administration

The compound of Example 2 was administered at a dose of 2 mg/kg with a volume of 5 mL/kg.

### 3. Procedure

The compound of Example 2 was intragastrically administered to the beagles. 0.5 mL of blood samples were collected from the forelimb vein 0 h before administration and 15 min, 30 min, 1.0 h, 2.0 h, 4.0 h, 6.0 h, 8.0 h, 11.0 h, and 24.0 h after administration. The blood samples were placed into EDTA-K2 anticoagulation test tubes and centrifuged at 10,000 rpm for 2 min (4 °C), and plasma was separated within 1 h and stored in a -80 °C refrigerator/dry ice before analysis. Two hours after administration, feeding was resumed. After the administration of the compound of Example 2, the plasma concentrations of the compound of Example 2 in the beagles were determined: 50 µL of the beagle plasma at each time point after the administration was taken, and 25 µL of internal standard solution (1 µg/mL camptothecin) and 450 µL of acetonitrile were added, and the mixture was vortexed and centrifuged at 3700 rpm for 10 min. 0.5 µL of supernatant was collected for LC/MS/MS analysis.

### 4. Pharmacokinetic parameters

**Table 3. Pharmacokinetic parameters of the compound of the present disclosure**

| Compound | Plasma concentration Cₘₐₓ (ng /mL) | Area under curve AUC₀₋ₜ (ng /mL*h) | Halflife T_{1/2} (h) | Residence time MRT (h) | Clearance rate CL/F (mL/min/kg) | Apparent volume of distribution Vz/F (mL/kg) |
|---|---|---|---|---|---|---|
| Example 2 | 2937 | 51585 | 12.5 | 19.4 | 0.48 | 490 |

Conclusion: the compound of the present disclosure has good pharmacokinetic absorption activity in dogs.

## Claims

1. A compound of general formula (I) or a pharmaceutically acceptable salt thereof: 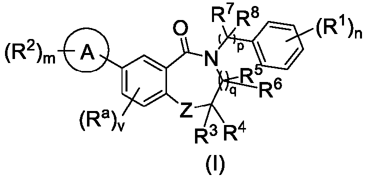 wherein:
ring A is selected from the group consisting of heteroaryl, cycloalkyl, heterocyclyl and polycyclic aryl;
Z is selected from the group consisting of -C(O)-, -S(O)ᵣ, -NRⁿ¹-, -CR^{z1}R^{z2}- and -O-;
each R¹ is identical or different and is independently selected from the group consisting of halogen, alkyl, alkenyl, alkynyl, nitro, cyano, -NRⁿ²Rⁿ³, -C(O)NRⁿ⁴Rⁿ⁵, -NRⁿ⁶C(O)R^{9a}, -C(O)R^{9b}, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
each R² is identical or different and is independently selected from the group consisting of halogen, alkyl, alkenyl, alkynyl, nitro, cyano, oxo, -NRⁿ²Rⁿ³, -C(O)NRⁿ⁴Rⁿ⁵, - NRⁿ⁶C(O)R^{9a}, -C(O)R^{9b}, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
each R^{a} is identical or different and is independently selected from the group consisting of halogen, alkyl, alkenyl, alkynyl, nitro, cyano, -NRⁿ²Rⁿ³, -C(O)NRⁿ⁴Rⁿ⁵, -NRⁿ⁶C(O)R^{9a}, -C(O)R^{9b}, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
R³ and R⁴ are identical or different and are each independently selected from the group consisting of a hydrogen atom, a deuterium atom, halogen, hydroxy, carboxyl, alkyl, haloalkyl, hydroxyalkyl, alkenyl, alkynyl, nitro, cyano, -NRⁿ²Rⁿ³, -C(O)NRⁿ⁴Rⁿ⁵, - C(O)R^{9b}, -OC(O)R^{9c}, -C(O)OR^{10a}, -OR^{10b}, cycloalkyl, heterocyclyl, aryl and heteroaryl; or R³ and R⁴, together with the carbon atom to which they are attached, form cycloalkyl or heterocyclyl;
R⁵ and R⁶ are identical or different and are each independently selected from the group consisting of a hydrogen atom, a deuterium atom, halogen, hydroxy, carboxyl, alkyl, haloalkyl, hydroxyalkyl, alkenyl, alkynyl, nitro, cyano, -NRⁿ²Rⁿ³, -OR^{10b}, cycloalkyl, heterocyclyl, aryl and heteroaryl;
R⁷ and R⁸ are identical or different and are each independently selected from the group consisting of a hydrogen atom, a deuterium atom, halogen, hydroxy, carboxyl, alkyl, haloalkyl, hydroxyalkyl, alkenyl, alkynyl, nitro, cyano, -NRⁿ²Rⁿ³, -OR^{10b}, cycloalkyl, heterocyclyl, aryl and heteroaryl; or R⁷ and R⁸, together with the carbon atom to which they are attached, form cycloalkyl or heterocyclyl;
R^{z1} and R^{z2} are identical or different and are each independently selected from the group consisting of a hydrogen atom, halogen, hydroxy, carboxyl, alkyl, haloalkyl, hydroxyalkyl, alkenyl, alkynyl, nitro, cyano, -NRⁿ²Rⁿ³, -C(O)NRⁿ⁴Rⁿ⁵, -C(O)R^{9b}, - OC(O)R^{9c}, -C(O)OR^{10a}, -OR^{10b}, cycloalkyl, heterocyclyl, aryl and heteroaryl; or R^{z1} and
R^{z2}, together with the carbon atom to which they are attached, form cycloalkyl or heterocyclyl;
Rⁿ¹, Rⁿ², Rⁿ³ , Rⁿ⁴ , Rⁿ⁵ , Rⁿ⁶ , Rⁿ⁷ and Rⁿ⁸ are identical or different at each occurrence and are each independently selected from the group consisting of a hydrogen atom, alkyl, haloalkyl, hydroxyalkyl, cycloalkyl, heterocyclyl, aryl and heteroaryl; or (Rⁿ² and Rⁿ³), (Rⁿ⁴ and Rⁿ⁵), and (Rⁿ⁷ and Rⁿ⁸), together with the nitrogen atom to which they are attached, each form heterocyclyl, wherein each heterocyclyl is independently optionally substituted with one or more substituents selected from the group consisting of halogen, oxo, alkyl, alkoxy, haloalkyl, haloalkoxy, cyano, amino, nitro, hydroxy, hydroxyalkyl, cycloalkyl, heterocyclyl, aryl and heteroaryl;
R^{9a}, R^{9b}, R^{9c}, R^{10a}, R^{10b} and R^{10c} are identical or different at each occurrence and are each independently selected from the group consisting of a hydrogen atom, alkyl, hydroxyalkyl, cycloalkyl, heterocyclyl, aryl and heteroaryl, wherein the alkyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
m is 0, 1, 2, 3, 4, 5 or 6;
n is 2, 3, 4 or 5;
p is 0, 1 or 2;
q is 1, 2 or 3;
r is 0, 1 or 2;
t is 0, 1 or 2; and
v is 0, 1, 2 or 3.

2. The compound of general formula (I) or the pharmaceutically acceptable salt thereof according to claim 1, wherein q is 1.

3. The compound of general formula (I) or the pharmaceutically acceptable salt thereof according to claim 1 or 2, being a compound of general formula (II) or a pharmaceutically acceptable salt thereof: 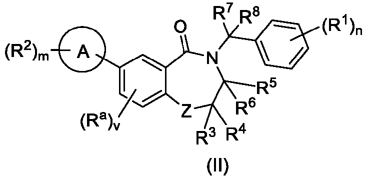 wherein:
ring A, R¹ to R⁸, R^{a}, v, Z, n and m are as defined in claim 1.

4. The compound of general formula (I) or the pharmaceutically acceptable salt thereof according to any one of claims 1 to 3, wherein R⁵ and R⁶ are both hydrogen atoms.

5. The compound of general formula (I) or the pharmaceutically acceptable salt thereof according to any one of claims 1 to 4, being a compound of general formula (III) or a pharmaceutically acceptable salt thereof: 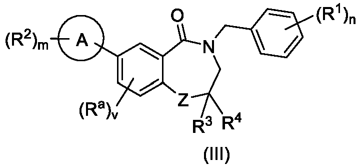 wherein:
ring A, R¹ to R⁴, R^{a}, v, Z, n and m are as defined in claim 1.

6. The compound of general formula (I) or the pharmaceutically acceptable salt thereof according to any one of claims 1 to 5, wherein R³ and R⁴ are both hydrogen atoms.

7. The compound of general formula (I) or the pharmaceutically acceptable salt thereof according to any one of claims 1 to 6, wherein n is 2; and two R¹ are identical or different and are each independently halogen or C₁₋₆ haloalkoxy.

8. The compound of general formula (I) or the pharmaceutically acceptable salt thereof according to any one of claims 1 to 6, being a compound of general formula (IV) or a pharmaceutically acceptable salt thereof: 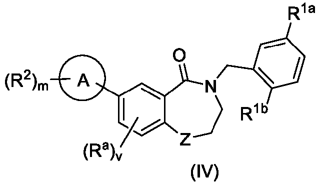 wherein:
R^{1a} and R^{1b} are identical or different and are each independently selected from the group consisting of halogen, alkyl, alkenyl, alkynyl, nitro, cyano, -NRⁿ²Rⁿ³, -C(O)NRⁿ⁴Rⁿ⁵, - NRⁿ⁶C(O)R^{9a}, -C(O)R^{9b}, -OC(O)R^{9c}, -C(O)OR^{10a}, -OR^{10b}, -S(O)ₜR^{10c}, cycloalkyl, heterocyclyl, aryl and heteroaryl, wherein the alkyl, alkenyl, alkynyl, cycloalkyl, heterocyclyl, aryl and heteroaryl are each independently optionally substituted with one or more substituents selected from the group consisting of halogen, oxo, alkyl, haloalkyl, alkoxy, haloalkoxy, nitro, cyano, -NRⁿ⁷Rⁿ⁸, hydroxy, hydroxyalkyl, cycloalkyl, heterocyclyl, aryl and heteroaryl;
ring A, R², R^{a}, v, Z, Rⁿ², Rⁿ³, Rⁿ⁴, Rⁿ⁵, Rⁿ⁶, Rⁿ⁷, Rⁿ⁸, R^{9a}, R^{9b}, R^{9c}, R^{10a}, R^{10b}, R^{10c}, t and m are as defined in claim 1.

9. The compound of general formula (I) or the pharmaceutically acceptable salt thereof according to claim 8, wherein R^{1a} and R^{1b} are identical or different and are each independently halogen or C₁₋₆ haloalkoxy.

10. The compound of general formula (I) or the pharmaceutically acceptable salt thereof according to any one of claims 1 to 9, wherein ring A is selected from the group consisting of 5- or 6-membered monocyclic heteroaryl, 8- to 10-membered polycyclic heteroaryl and 8- to 10-membered polycyclic aryl; preferably, ring A is selected from the group consisting of pyridinyl, benzimidazolyl, benzoxazolyl, benzothiazolyl, pyrrolotriazinyl, 5,6,7,8-tetrahydro-triazolopyrazinyl, imidazopyridazinyl and [1,2,4]triazolo[1,5-a]pyridinyl; and more preferably, ring A is [1,2,4]triazolo[1,5-a]pyridinyl.

11. The compound of general formula (I) or the pharmaceutically acceptable salt thereof according to any one of claims 1 to 10, wherein R² is -NRⁿ²Rⁿ³, Rⁿ² and Rⁿ³ are identical or different and are each independently a hydrogen atom or C₁₋₆ alkyl, and m is 1.

12. The compound of general formula (I) or the pharmaceutically acceptable salt thereof according to any one of claims 1 to 11, wherein Z is -CH₂- or -O-.

13. The compound of general formula (I) or the pharmaceutically acceptable salt thereof according to any one of claims 1 to 12, wherein each R^{a} is identical or different and is independently halogen or C₁₋₆ alkyl, and v is 0, 1 or 2.

14. The compound of general formula (I) or the pharmaceutically acceptable salt thereof according to any one of claims 1 to 13, being selected from the group consisting of the following compounds:

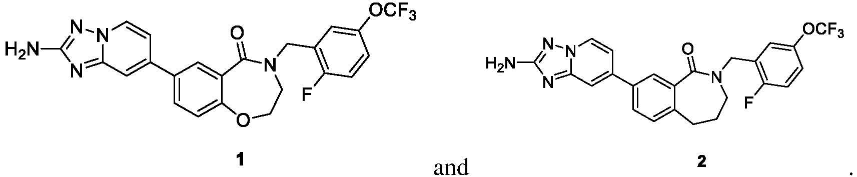

15. A compound of general formula (IA) or a salt thereof: 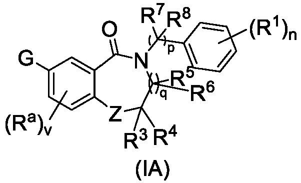 wherein:
G is 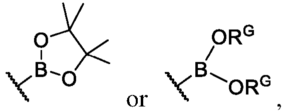 and R^{G} is a hydrogen atom or C₁₋₆ alkyl;
R¹, R³ to R⁸, R^{a}, v, Z, p, q and n are as defined in claim 1.

16. The compound of general formula (IA) or the salt thereof according to claim 15, being selected from the group consisting of the following compounds:

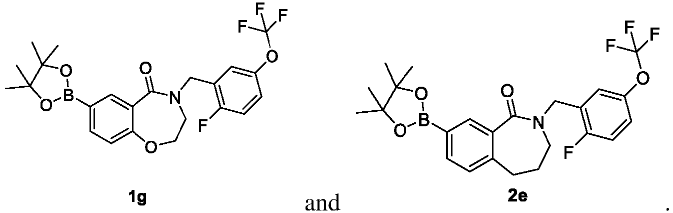

17. A method for preparing a compound of general formula (I) or a pharmaceutically acceptable salt thereof, wherein the method comprises the following step: 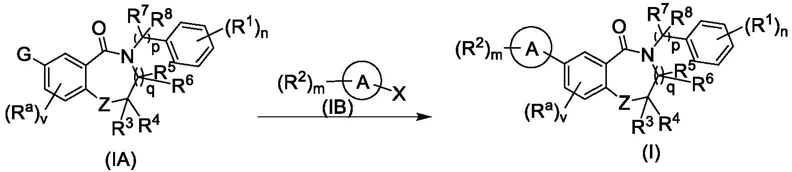 conducting a coupling reaction of a compound of general formula (IA) or a salt thereof with a compound of general formula (IB) or a salt thereof to give the compound of general formula (I) or the pharmaceutically acceptable salt thereof,
wherein:
G is 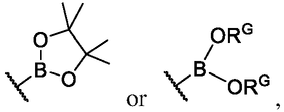 and R^{G} is a hydrogen atom or C₁₋₆ alkyl;
X is halogen, preferably Br;
ring A, R¹ to R⁸, R^{a}, v, Z, p, q, m and n are as defined in claim 1.

18. A pharmaceutical composition, wherein the pharmaceutical composition comprises the compound of general formula (I) or the pharmaceutically acceptable salt thereof according to any one of claims 1 to 14, and one or more pharmaceutically acceptable carriers, diluents or excipients.

19. Use of the compound of general formula (I) or the pharmaceutically acceptable salt thereof according to any one of claims 1 to 14 or the pharmaceutical composition according to claim 18 in the preparation of a medicament for inhibiting RIPK1 kinase.

20. Use of the compound of general formula (I) or the pharmaceutically acceptable salt thereof according to any one of claims 1 to 14 or the pharmaceutical composition according to claim 18 in the preparation of a medicament for treating and/or preventing a RIPK1 kinase-mediated disease or disorder.

21. The use according to claim 20, wherein the disease or disorder is selected from the group consisting of a CNS disease, an autoimmune disease, an inflammatory disease, an ischemic disease and a cancer; preferably, the CNS disease is a neurodegenerative disease.

22. Use of the compound of general formula (I) or the pharmaceutically acceptable salt thereof according to any one of claims 1 to 14 or the pharmaceutical composition according to claim 18 in the preparation of a medicament for treating and/or preventing amyotrophic lateral sclerosis, multiple sclerosis, Alzheimer's disease, Huntington's chorea, Friedreich's ataxia, Parkinson's disease, spinal muscular atrophy, stroke, human immunodeficiency virus-associated dementia, autism, schizophrenia, rheumatoid arthritis, osteoarthritis, ankylosing spondylitis, systemic onset juvenile idiopathic arthritis, psoriasis, dermatitis, systemic lupus erythematosus, systemic inflammatory response syndrome, pancreatitis, encephalitis, nonalcoholic steatohepatitis, alcoholic steatohepatitis, autoimmune hepatitis, autoimmune hepatobiliary diseases, primary sclerosing cholangitis, nephritis, ulcerative colitis, Crohn's disease, retinal degenerative diseases, retinal detachment, retinitis pigmentosa, macular degeneration, pancreatitis, Sjögren's syndrome, systemic scleroderma, ischemia-reperfusion injury of solid organs, cerebral ischemia, ischemic heart disease, acute kidney injury, ischemic brain injury, sepsis, diabetes and atherosclerosis; preferably, in the preparation of a medicament for treating and/or preventing amyotrophic lateral sclerosis, multiple sclerosis, Alzheimer's disease, Huntington's chorea, Friedreich's ataxia, Parkinson's disease and spinal muscular atrophy.

23. The use according to claim 21, wherein the cancer is selected from the group consisting of leukemia, lymphoma, macroglobulinemia, heavy chain disease, sarcoma, carcinoma, pancreatic cancer, breast cancer, ovarian cancer, prostate cancer, squamous cell carcinoma, sweat gland carcinoma, sebaceous carcinoma, papillary carcinoma, cystadenocarcinoma, medullary carcinoma, bronchogenic carcinoma, fiver cancer, cholangiocarcinoma, choriocarcinoma, seminoma, embryonal carcinoma, Wilms tumor, cervical cancer, endometrial cancer, testicular cancer, lung cancer, bladder cancer, neuroglioma, medulloblastoma, craniopharyngioma, ependymoma, pinealoma, hemangioblastoma, acoustic neuroma, schwannoma, neurofibroma, retinoblastoma, melanoma, skin cancer, kidney cancer, nasopharyngeal cancer, gastric cancer, esophageal cancer, head and neck cancer, colorectal cancer, small intestine cancer, gallbladder cancer, pediatric tumor, urothelial cancer, ureteral tumor, thyroid cancer, osteoma, neuroblastoma, brain tumor and myeloma.
